# EUROPEAN PATENT APPLICATION

(11) **EP 4 036 226 A1**
(43) Date of publication of application: **03.08.2022**
(21) Application number: 21305138.6
(22) Date of filing: 02.02.2021
(51) Int. Cl.: C12N 7/00, A61K 39/12, C12Q 1/70

(54) **ATTENUATED AFRICAN SWINE FEVER VIRUS AND ITS USE AS A VACCINE**

(71) Applicant: Agence Nationale chargée de la Sécurité Sanitaire de l'Alimentation, de l'Environnement et du Travail, 94700 Maisons-Alfort (FR)
(72) Inventor: BLOT LE POTIER, Marie-Frédérique, 22590 Pordic (FR); BOURRY, Olivier, 22400 Lamballe (FR); HUTET, Evelyne, 22190 Plérin (FR); LE DIMNA, Mireille, 22440 Ploufragan (FR); CHASTAGNER, Amélie, 42670 Ecoche (FR); PABOEUF, Frédéric, 22440 Ploufragan (FR); BLANCHARD, Yannick, 35580 Goven (FR); LUCAS, Pierrick, 22120 Yffiniac (FR)
(74) Representative: Novagraaf Technologies

(57) **Abstract**

The present invention relates to an attenuated African Swine Fever (ASF) virus, wherein :
• genes MGF 360-12L, 360-13L, 360-14L, 505-2R, 505-3R are deleted or are interrupted or mutated such that the genes are not transcribed and/or translated,
• ORF of ASFV_G_ACD_00520 is deleted or is interrupted or mutated such that it is not transcribed and/or translated, and
• genes MGF 505-1R et 505-4R are truncated,
compared to the genome of the corresponding unattenuated virus.

The present invention also refers to a vaccine comprising the attenuated ASF virus, and its use in preventing African Swine Fever in a subject.

The present invention also relates to an in-vitro method for obtaining the attenuated ASF virus, which comprises at least one step of thermal-attenuation of a virulent ASFV virus strain selected among Georgia 2007/1, Pig/HLJ/2018, a strain of ASF virus of genotype II or a genetically close ASF virus strain, and amplification by inoculation of Specific-Pathogen-Free pigs and selecting said attenuated ASF virus.

The present invention refers to an in vitro method for the differential detection of the attenuated ASF virus and of the corresponding non-attenuated ASF virus as well.

SEQUENCE LISTING

<110>  ANSES

<120>  Attenuated African Swine Fever virus and its use as a vaccine

<130>  BNT229559EP00

<160>  9

<170>  PatentIn version 3.5

<210>  1
<211>  2126
<212>  DNA
<213>  African swine fever virus

<400>  1
atgttctctc tccagaactt atgtcgaaaa acattaccta accgtaaact tcctgaattt      60

tttgacgaat atatattaca actgctggga ttatactggg aaaaccatgg aactattcaa     120

cgagcaggaa acaactgtgt gcttatacag caacataccc tcattcccgt aaatgaagcc     180

ctgagaacag cagcatctga agaaaattat gagatcgtga gccttttatt agcgtgggag     240

gggaaccttt actatgctat tataggggct ctagagggca accgccacga cttaattcgt     300

aaatatgatg accaaatcaa ggaccatcat gaaattctgc cattcattga cgatccagtc     360

atatttcaca aatgccatat catgcggcaa tgcttttttg attgtatttt atatcaagct     420

gtaaaatata gtaagtttcg cgttcttctt tactttaaac atagattaga ggatgatttg     480

cccttcactc atttacttat tgaaaaggca tgtaaagatc ataattatga agttattaaa     540

tggatatatg aaaacctaca tatctacaat atgatagata cctttgaatg tgctattgcc     600

cataaggatc tacatctata ttgtttgggg tatagattta tatataacag aatcgtaccc     660

gataagtatc atcatttaga tattcgcatg ctttcaagcc tacaactcct acataaggtg     720

gcagccaaag gatcgctgtg tttgtacagc gaaacatcgt cctttctacc aatgaggccc     780

tgagaatcgc agcctcagag ggaaacgaaa gggtaataaa acttctgtta tcatgggagg     840

gaaattttca ttatgtgatc ataggagctc tagagggtga ccaatatgac ctaattcata     900

agtatgatag tcaaattaaa gactaccaca tgattttatc attgatccaa aatgcaaata     960

cctttgaaaa gtgtcatcag ttatccaata gtaatatgtg gtgtcttata cagaatgcta    1020

taaaatataa tatgctccct attctccaaa aacacagaaa tattctgaca catgagggag    1080

agaatcagga attgtttgag atggcatgtg aggaacagaa atatgacata gttttatgga    1140

taggacaaac cctaatgtta aatgagccgg agtttatttt tgatatcgcc ttcgaacgga    1200

tagatttttc tttattaaca atgggttata gccttctttt tgataacaag atgagtagta    1260

tagacattca tgatgaagaa gatcttactt cattaccaac agaacacctc gaaaaagcag    1320

ccactaaggg atgtttcttc tttatgctag aaactttaaa acatggtgga aatgtaaata    1380

tggcagtctt atctaaagct gttgagtata atcatagaaa aattttagac cattttattc 1440

ggcggcaaaa atgtttatca cgtgaagaga ttgaaaacct attattaacc gccataacca 1500

attgtgcatc cataaaaacg ttaaacttac tcttgtctta cctaaactat tccgtaaaaa 1560

atatcattgg aaaaatagta caacatgtca taaaagatgg tgattatacc atcatattac 1620

ttttaaaaaa aaagaaaata aacctagtgg aacctgtttt aacaggtttt atagattatt 1680

actatagcta ttgttttata aaacatttta tccaagagtt tgctattcgt ccggaaaaac 1740

tgattaaaat ggccgcgcga aaaggtaaac taaatatgat tatcgaattc cttaacgaaa 1800

aatatgttca taaagatgat cttggaacta tatttaaata tctcaaaacc ctagtatgta 1860

ccatgaaaca taaaaaagga aaagagacat taattgttct tattcataaa atatatcaag 1920

atattcatct ggagactaaa gaaaaattta aattattaag attttatgtc atgcatgatg 1980

caactatcca atttctatct atgtgcaaag actgttttaa tttagccggt tttaaaccat 2040

ttgttttaga atgtttggat attgctatta aaaaaaatta ccctgatatg atacaatata 2100

tagaaattct atcgaaatct gagtaa 2126

<210> 2
<211> 7458
<212> DNA
<213> African swine fever virus

<400> 2
acttagattt tatcctagaa accttaaagt atgatcataa taaagataat ataaatatta 60

ttctaacaca agctgcaacc tataaccata gaaaaatttt aatctatttc attcctcaat 120

caacccacgc acagatagaa caatgtttac tagtggcgat aaaagcaaaa tcttccagga 180

aaaccttgaa cttactactg tctcacctaa acctttccat caacctcatc aaaaaaataa 240

gccattatgt tgccacttac aattcaacaa atataatagg cattctgagt atgcggcgga 300

aaaagaagat atatttagat atcatattga caaaatttgt aaaaaaagct atttttaata 360

agtttgtcgt tcgatgtatg gatacatttt ctataaaccc ggaaagaatc cttaaaatag 420

ccgcgcgaat aaataggatg atgttagtga aaaaaatatc tgaacatgtt tggaaaaatc 480

atgcggttag acttaaatac cttaaacatg cggtacacac gatgaagcat aaagatggga 540

aaaatagact catgaacttt atctatgatc gctgttatta ccatatgcaa ggggaagaaa 600

tctttagcct cgcaagattt tatgcaatcc atcatgcacc aaagttgttt gacgtttttt 660

atgattgttg tatcctagat acgatacgat tcaaaagcct tcttttagat tgttcacata 720

tcataggtaa aaacgctcat gatgctacca atatcaacat cgtgaacaag tatatcggca 780

acctgtttgt tatgggagtt cttagcaaaa aagaaatctt acaggactat ccatccattt 840

attctaaaca atacatgcct tagtttattt tttttgcggc cgaaacatta ttcttaccct 900

agaaaacgct tatagtcatc ttaaatcata ggtaaggaag atcatcatat tttttgaaac 960

gtaatttttt aacgcatgat ctatgatttc agggtccgtg cttttaggca acggggtggt 1020

ggccggacta taaatcttta gggataaaat gttctttata agctcatacc cttcccctaa 1080

agctgtagta ccctcttcga aaacatcagc ccccagatct atacaaaaga acatgttttc 1140

tatattatag tactgtattg agctaagcat ggcttgattg atgttggcgc ccaggacata 1200

gcagtagtac atggttgaaa ggttgtggtc tttgatgcag gcgatccgca tcatctcttc 1260

tatgtccata tggatcttgt ccttttcata cgcctcatga aggtcaaaca cattattaaa 1320

acaaagagca catgttaacc gccacgtatt caggtgtgta tatttttggt aaaaatactg 1380

tatggcctct ttcaggttat agcgtatggc tatagcgtac cagtatttga gtagtaatgt 1440

actgagcgaa aactcattat ttagcagatc ggtttttact attaactccc ttaactccca 1500

gaaaatttct atcctcattt ttatattatt tactttttgt aatatcggat tgttggaaaa 1560

cacctcatgg cataaaataa tgttactact agttttatga aactttagat ctataaaaat 1620

ttgtaaaatt tcttcttcat tcaaggtttc cttggcacct agctctcgac agaggtccca 1680

ggtgtgctcc gtgttgacag ataccagccc gtagttgatg tccgcccccc actctgcaaa 1740

cagttttata aggttgtagt tgttttccct tacagccttc actaacgccg tatttaggtt 1800

taagccctct ttaatacctg ctgattttat gagccttagg ttatgatcaa acgtgatcgg 1860

agcatcatgc caccataggt cataacactt taaaagataa tgttggttcg tgggcacgca 1920

ttgtccagcc aacacctttt tggtcagaga ttgcagggaa ggcaacatgt ctcttcatct 1980

tttaaaaaaa aatcaaatta attagccgaa taaatttttc tttcgagggc tttttaaaag 2040

agctctttaa gagctcttta agagcttttt aagagattaa aaaattattc ttgctggcat 2100

tctgccaagt atgcggcatt cctatcatct atagtatatt atgagaatat tcccaaatga 2160

tggataagtt ttttgattta taatctttta ataaactgct tatttcttcg ggtcctttta 2220

agtttagtgg caaggaagca tctgagctgt aaatatccaa agccaaacta tggctcagaa 2280

aattataacc tttttgttcc gctatggcac gaccctcttc aaaggcatta ccacccaaat 2340

ctatacagaa aaatatatta ccgatgttat aatattgtac tgaagtaagc atagcttggt 2400

tgatgttgcc ccccagcgcg taacagtaat atattgttaa tggattgtta tccttggtag 2460

aagccagaca tatcatgtca tggacgtcta tttggatgtt ttccttgtgg tacatctcat 2520

gaagctcata tattttgtta taatacagga gacattttaa tcgccattca ttaagatccg 2580

tatatttctc atctagaaaa caaatggcgt ccttacaatc gtattgtact gctttggcgt 2640

accaatactt cactagtaaa ccatttaact cgtccgtttc ttttatttct atgagccccc 2700

atagtctttt ataaattaag ccccttaatt gtataacaaa tttgttttct aaaataggat 2760

tattcataaa aatttcatgg cacaaaataa tactgccgct ggttttattg tgcattatcc 2820
tggtaaaaat acggaaaata tcgttgtcct ctagagtttc tttggcgcct agctgtctac 2880
acaactctcg gatgtgcttc gtattgatag aaagcaaacc atagttgata tttgcgcccc 2940
actctgtaaa gagctttatc agactatagt tgttttcctt aacagctatt attaatgcca 3000
cacgaaggtc tatatcttct cctaaaaatc ctgattttat ttgtattcgg ccacgatcca 3060
tacaaagctt gagaggagca tcatgccacc ataggccaca atatttcaaa atgcagtgtt 3120
catctattga caaacactgg ctggctatcg tctttttgac gagggtctgc agagagagcg 3180
gcaacgacat gtttcttttt caccaaaaaa aaatcaaatg ttctcgtctt taaaggttaa 3240
ttcatgttct taaaatgttc atttcatgat agtgattaat aatatggttt aataacgcta 3300
gaaggcttgt ttataagaca gtcataagca gtctataaga cagtctataa gcagtctata 3360
agacagtcta tgacttagtc tataactata atttctggat gggctgtaag atactcttcg 3420
gctcgtttca gattttttga agtatatgtc tttagcatat catatatttc ctggggttcg 3480
gttacatcta ataccaaggt cacatcacgg ctgaaaagct gctttactaa gaaaatgttg 3540
ctcaagttat acatataagc tttgtgcgca atgagttgtg ccctatcaaa atcggcagcc 3600
cccaaatcaa tacagaaaaa catgtttaaa gtattattgt tatagataga aagattcatg 3660
ccataatcga gactagcccc caacctatga cagtaataaa tggccgcgta atttttttcc 3720
cgcaagcaag caaatttcat catcagatta gggctgatgc aaatctcttt ttcacgacac 3780
aactcgtgta tgtcaaaaat gttattaaaa taaaggctac aagctacccg ccaatagagg 3840
tgatttttat gccttttata gaaatagtga atagcctttg taaaattatg tcgtaatgcc 3900
agggcaaacc aaaactttgt taataggtgg tgcgccgtat cccccgtcaa cggaatgttt 3960
gaacaggtgt acgtaactgt gtctaaagtg gttctagtta cggtttccaa gagtggatta 4020
tgacaaaaca tgtcataacc cagcagaact cctgcacagg attttagcct ggccacttct 4080
tttaaaattt ccagaagacg gggttcggat acaggcgtta agcctcccag ttccgcacac 4140
agccgcttta gatacacggc aggaacacgt ataagcccat attcaggatt tgcgccccaa 4200
tccacaaata aacgtataag ttcaagatta tcgctcttca cggcctttac tagcgccgct 4260
tcgagacaaa gatcatcctc agaaaaacac tgtaaatgtt tatacgaaaa aacttgctta 4320
caattgttac ataggtgaat aggacctaaa tcccaccaca aaccaaaacg ctgcaacgta 4380
taatcatagt cacttgaaag ataattgcat gccacaactt ttttggccaa cgtttgtaaa 4440
gacaacatac taagtttaaa acatcttaaa tctaagctag ctaactttca agaaaaccct 4500
ctatccctaa gaatatatct tataactaga cttatagcag taaaaatcaa ctttggttat 4560
tctttttaat ataaaacgtc taattacttg caaaggacta taaagcccat tttcctcagc 4620
tagaattttt attttttaat gaagtagggg gatatgtttt cccttcaaga cctttgccga 4680

aagcatcttt ttattcttcc cgatgttttt ggcgagcatg tactacaacg attaggactg 4740

tattggagat gtcacggctc ccttcaacgc ataggagacg accacatact catacgacgg 4800

gatctcatcc tttccaccaa cgaggcctta agaatggcgg gagaggaagg aaacaatgaa 4860

gtagtaaagc tcttgttact gtggaaggga aatcttcatt acgccgtcat aggagccttg 4920

cagggtgatc aatatgacct gatccataag tatgaaaacc aaatcggcga ctttcatttt 4980

atcttaccat tgattcaaga cgcgaatacg tttgaaaaat gccacgcttt agaacgtttt 5040

tgtggtgttt catgtctgct aaaacatgct acaaaataca acatgctccc tattctccaa 5100

aaataccaag aagagctgtc tatgagagcg tatcttcacg aaaccctatt tgaactagca 5160

tgcctatggc agaggtatga tgtccttaaa tggatagagc aaaccataca tgtttacgac 5220

ctaaagatta tgtttaatat tgccatctcc aagagggatc tgactatgta ctccttagga 5280

tatattttcc tttttgatag agggaacacc gaagctacgt tgctaacgca acatctcaag 5340

aagacagcgg ccaaagggct cctccacttt gtgctagaaa cgttaaaata cggcggcaac 5400

atagataccg tcctgaccca agccgtaaag tacaatcata gaaaactttt agattatttt 5460

ctgcgtcaac tacctcgtaa acatattgaa aaacttttgt tgctggccgt gcaggaaaag 5520

gcttctaaaa aaacattgaa cttactgttg tcacatttaa actactccgt gaaacgcatc 5580

aaaaaactac cgcgctatgt gatagagtac gagtccacct tggtgataaa gattttatta 5640

aaaaaaagag tgaacctgat agatgccatg ttggaaaaga tggtaagata tttttctgcg 5700

acgaaagtga ggacgatcat ggatgagctt tcgattagtc cggaaagagt cattaagatg 5760

gctatacaga aaatgagaac ggatatcgta atccatactt cttatgtttg ggaggatgat 5820

ctagaacgtc ttactcgtct taaaaatatg gtatacacca taaagtacga acatgggaaa 5880

aaaatgttaa ttaaagtcat gcacggcata tacaaaaact tattatacgg cgaaagggaa 5940

aaagtcatgt tttatttagc caagctctat gttgctcaaa acgcggccac ccaattcaga 6000

gacatttgta aggactgtta caaactggat gtggcacggt ttaaaccgcg gtttaagcaa 6060

ctaatattag actgtttaga aattattact aaaaaatctt gctatagtat cctggaaatc 6120

ttagaaaaac atattatttc cctgtttact atgaaagtta tgactgaaga agaaaaaaac 6180

ctatgtttag aaatattata taaagtaatt cattataaaa caatacaatg ttaaaattca 6240

atagatatcc atcattaata ttgattatat tttcgaatat tatcttctat ggtgcaagat 6300

aatcatctag cgcgtgaaac atgtcctctt ctcttcagga actttgtcga aaaaagctgc 6360

ctgactgcat acttccagag ttttttgacg actatgtatt gcaactgtta ggactgcact 6420

ggcaagatca tggttccctt cagcgtatcg agaagaacca gatacttgtt caacaggaac 6480

ccatccatat caatgaagca ctcaaagtag cagcatcgga agggaactat gaaatcgtag 6540

agctgttgtt gtcatggagg gcagatcccc gctacgccgt cgtaggagcc ctagaaagca 6600

aatactatga cctggtttac aaatactatg accaagttaa agactgccat gatatcttgc 6660

cgctgattca aaatccggaa acattcgaaa gatgtcatga gttaaacagc acctgttcac 6720

tgaaatgctt attcaagcat gctgtgataa atgacatgct gccgattctt caaaaatata 6780

cagactatct ggataggtgg gagtattgca gccagatgct gttcgaactg gcatgtagta 6840

aaaaaaaata tgagatggtt gtgtggatag agggagttct aggcgtcggc aaagttacat 6900

ctcttttcac cattgcgatt agcaacagag acctacagct gtattctctg ggctactcaa 6960

ttatccttga gaatttgtac tcctgtggac aggaccccaa gtttttacta aatcatttcc 7020

tgcgagacgt ttcaataaaa gggcttctac cctttgtaat caaaaccata gaatatggtg 7080

gaagcaagga gatagccata actctggcta aaaaatatca gcataaacat attttgaaat 7140

acttcgaaac ctgggaaagc taggttcagt atggtgtact cactattgta gtgaatcgta 7200

tcctgtaaat tttgtaaaaa agcttaaact tttgaccaca tcatattgtt ttagaaatct 7260

caaaccagtg aacaacagtc ttatcataca ttaaaattcc agtaaaattt atattttttt 7320

tggtaaacaa atgttttctc ttcaagacat ctgtcggaaa catctttttc aacttcctga 7380

cgcttttgat gaatatatat tacaagcgct aggactatac tgggaaaaac acggatctct 7440

tcaacgaata agaaagga 7458

<210> 3
<211> 9584
<212> DNA
<213> African swine fever virus

<400> 3
atgttctctc tccagaactt atgtcgaaaa acattaccta accgtaaact tcctgaattt 60

tttgacgaat atatattaca actgctggga ttatactggg aaaaccatgg aactattcaa 120

cgagcaggaa acaactgtgt gcttatacag caacataccc tcattcccgt aaatgaagcc 180

ctgagaacag cagcatctga agaaaattat gagatcgtga gccttttatt agcgtgggag 240

gggaaccttt actatgctat tataggggct ctagagggca accgccacga cttaattcgt 300

aaatatgatg accaaatcaa ggaccatcat gaaattctgc cattcattga cgatccagtc 360

atatttcaca aatgccatat catgcggcaa tgcttttttg attgtatttt atatcaagct 420

gtaaaatata gtaagtttcg cgttcttctt tactttaaac atagattaga ggatgatttg 480

cccttcactc atttacttat tgaaaaggca tgtaaagatc ataattatga agttattaaa 540

tggatatatg aaaacctaca tatctacaat atgatagata cctttgaatg tgctattgcc 600

cataaggatc tacatctata ttgtttgggg tatagattta tatataacag aatcgtaccc 660

gataagtatc atcatttaga tattcgcatg ctttcaagcc tacaactcct acataaggtg 720

gcagccaaag gatacttaga ttttatccta gaaaccttaa agtatgatca taataaagat 780

aatataaata ttattctaac acaagctgca acctataacc atagaaaaat tttaatctat 840

ttcattcctc aatcaaccca cgcacagata gaacaatgtt tactagtggc gataaaagca 900

aaatcttcca ggaaaacctt gaacttacta ctgtctcacc taaacctttc catcaacctc 960

atcaaaaaaa taagccatta tgttgccact tacaattcaa caaatataat aggcattctg 1020

agtatgcggc ggaaaaagaa gatatattta gatatcatat tgacaaaatt tgtaaaaaaa 1080

gctattttta ataagtttgt cgttcgatgt atggatacat tttctataaa cccggaaaga 1140

atccttaaaa tagccgcgcg aataaatagg atgatgttag tgaaaaaaat atctgaacat 1200

gtttggaaaa atcatgcggt tagacttaaa taccttaaac atgcggtaca cacgatgaag 1260

cataaagatg ggaaaaatag actcatgaac tttatctatg atcgctgtta ttaccatatg 1320

caaggggaag aaatctttag cctcgcaaga ttttatgcaa tccatcatgc accaaagttg 1380

tttgacgttt tttatgattg ttgtatccta gatacgatac gattcaaaag ccttctttta 1440

gattgttcac atatcatagg taaaaacgct catgatgcta ccaatatcaa catcgtgaac 1500

aagtatatcg gcaacctgtt tgttatggga gttcttagca aaaaagaaat cttacaggac 1560

tatccatcca tttattctaa acaatacatg ccttagttta ttttttttgc ggccgaaaca 1620

ttattcttac cctagaaaac gcttatagtc atcttaaatc ataggtaagg aagatcatca 1680

tattttttga aacgtaattt tttaacgcat gatctatgat ttcagggtcc gtgcttttag 1740

gcaacggggt ggtggccgga ctataaatct ttagggataa aatgttcttt ataagctcat 1800

acccttcccc taaagctgta gtaccctctt cgaaaacatc agcccccaga tctatacaaa 1860

agaacatgtt ttctatatta tagtactgta ttgagctaag catggcttga ttgatgttgg 1920

cgcccaggac atagcagtag tacatggttg aaaggttgtg gtctttgatg caggcgatcc 1980

gcatcatctc ttctatgtcc atatggatct tgtccttttc atacgcctca tgaaggtcaa 2040

acacattatt aaaacaaaga gcacatgtta accgccacgt attcaggtgt gtatattttt 2100

ggtaaaaata ctgtatggcc tctttcaggt tatagcgtat ggctatagcg taccagtatt 2160

tgagtagtaa tgtactgagc gaaaactcat tatttagcag atcggttttt actattaact 2220

cccttaactc ccagaaaatt tctatcctca tttttatatt atttactttt tgtaatatcg 2280

gattgttgga aaacacctca tggcataaaa taatgttact actagtttta tgaaacttta 2340

gatctataaa aatttgtaaa atttcttctt cattcaaggt ttccttggca cctagctctc 2400

gacagaggtc ccaggtgtgc tccgtgttga cagataccag cccgtagttg atgtccgccc 2460

cccactctgc aaacagtttt ataaggttgt agttgttttc ccttacagcc ttcactaacg 2520

ccgtatttag gtttaagccc tctttaatac ctgctgattt tatgagcctt aggttatgat 2580

caaacgtgat cggagcatca tgccaccata ggtcataaca ctttaaaaga taatgttggt 2640

tcgtgggcac gcattgtcca gccaacacct ttttggtcag agattgcagg gaaggcaaca 2700

tgtctcttca tcttttaaaa aaaaatcaaa ttaattagcc gaataaattt ttctttcgag 2760

ggctttttaa aagagctctt taagagctct ttaagagctt tttaagagat taaaaaatta 2820

ttcttgctgg cattctgcca agtatgcggc attcctatca tctatagtat attatgagaa 2880

tattcccaaa tgatggataa gttttttgat ttataatctt ttaataaact gcttatttct 2940

tcggggtcct ttaagtttag tggcaaggaa gcatctgagc tgtaaatatc caaagccaaa 3000

ctatggctca gaaaattata acctttttgt tccgctatgg cacgaccctc ttcaaaggca 3060

ttaccaccca aatctataca gaaaaatata ttaccgatgt tataatattg tactgaagta 3120

agcatagctt ggttgatgtt gccccccagc gcgtaacagt aatatattgt taatggattg 3180

ttatccttgg tagaagccag acatatcatg tcatggacgt ctatttggat gttttccttg 3240

tggtacatct catgaagctc atatattttg ttataataca ggagacattt taatcgccat 3300

tcattaagat ccgtatattt ctcatctaga aaacaaatgg cgtccttaca atcgtattgt 3360

actgctttgg cgtaccaata cttcactagt aaaccattta actcgtccgt ttcttttatt 3420

tctatgagcc cccatagtct tttataaatt aagcccctta attgtataac aaatttgttt 3480

tctaaaatag gattattcat aaaaatttca tggcacaaaa taatactgcc gctggtttta 3540

ttgtgcatta tcctggtaaa aatacggaaa atatcgttgt cctctagagt ttctttggcg 3600

cctagctgtc tacacaactc tcggatgtgc ttcgtattga tagaaagcaa accatagttg 3660

atatttgcgc cccactctgt aaagagcttt atcagactat agttgttttc cttaacagct 3720

attattaatg ccacacgaag gtctatatct tctcctaaaa atcctgattt tatttgtatt 3780

cggccacgat ccatacaaag cttgagagga gcatcatgcc accataggcc acaatatttc 3840

aaaatgcagt gttcatctat tgacaaacac tggctggcta tcgtcttttt gacgagggtc 3900

tgcagagaga gcggcaacga catgtttctt tttcaccaaa aaaaaatcaa atgttctcgt 3960

ctttaaaggt taattcatgt tcttaaaatg ttcatttcat gatagtgatt aataatatgg 4020

tttaataacg ctagaaggct tgtttataag acagtcataa gcagtctata agacagtcta 4080

taagcagtct ataagacagt ctatgactta gtctataact ataatttctg gatgggctgt 4140

aagatactct tcggctcgtt tcagattttt tgaagtatat gtctttagca tatcatatat 4200

ttcctggggt tcggttacat ctaataccaa ggtcacatca cggctgaaaa gctgctttac 4260

taagaaaatg ttgctcaagt tatacatata agctttgtgc gcaatgagtt gtgccctatc 4320

aaaatcggca gcccccaaat caatacagaa aaacatgttt aaagtattat tgttatagat 4380

agaaagattc atgccataat cgagactagc cccaacctta tgacagtaat aaatggccgc 4440

gtaatttttt tcccgcaagc aagcaaattt catcatcaga ttagggctga tgcaaatctc 4500

tttttcacga cacaactcgt gtatgtcaaa aatgttatta aaataaaggc tacaagctac 4560

ccgccaatag aggtgatttt tatgcctttt atagaaatag tgaatagcct ttgtaaaatt 4620

atgtcgtaat gccagggcaa accaaaactt tgttaatagg tggtgcgccg tatcccccgt 4680

caacggaatg tttgaacagg tgtacgtaac tgtgtctaaa gtggttctag ttacggtttc 4740

caagagtgga ttatgacaaa acatgtcata acccagcaga actcctgcac aggattttag 4800

cctggccact tcttttaaaa tttccagaag acggggttcg gatacaggcg ttaagcctcc 4860

cagttccgca cacagccgct ttagatacac ggcaggaaca cgtataagcc catattcagg 4920

atttgcgccc caatccacaa ataaacgtat aagttcaaga ttatcgctct tcacggcctt 4980

tactagcgcc gcttcgagac aaagatcatc ctcagaaaaa cactgtaaat gtttatacga 5040

aaaaacttgc ttacaattgt tacataggtg aataggacct aaatcccacc acaaaccaaa 5100

acgctgcaac gtataatcat agtcacttga aagataattg catgccacaa cttttttggc 5160

caacgtttgt aaagacaaca tactaagttt aaaacatctt aaatctaagc tagctaactt 5220

tcaagaaaac cctctatccc taagaatata tcttataact agacttatag cagtaaaaat 5280

caactttggt tattcttttt aatataaaac gtctaattac ttgcaaagga ctataaagcc 5340

cattttcctc agctagaatt tttatttttt aatgaagtag ggggatatgt tttcccttca 5400

agacctttgc cgaaagcatc tttttattct tcccgatgtt tttggcgagc atgtactaca 5460

acgattagga ctgtattgga gatgtcacgg ctcccttcaa cgcataggag acgaccacat 5520

actcatacga cgggatctca tcctttccac caacgaggcc ttaagaatgg cgggagagga 5580

aggaaacaat gaagtagtaa agctcttgtt actgtggaag ggaaatcttc attacgccgt 5640

cataggagcc ttgcagggtg atcaatatga cctgatccat aagtatgaaa accaaatcgg 5700

cgactttcat tttatcttac cattgattca agacgcgaat acgtttgaaa aatgccacgc 5760

tttagaacgt ttttgtggtg tttcatgtct gctaaaacat gctacaaaat acaacatgct 5820

ccctattctc caaaaatacc aagaagagct gtctatgaga gcgtatcttc acgaaaccct 5880

atttgaacta gcatgcctat ggcagaggta tgatgtcctt aaatggatag agcaaaccat 5940

acatgtttac gacctaaaga ttatgtttaa tattgccatc tccaagaggg atctgactat 6000

gtactcctta ggatatattt tcctttttga tagagggaac accgaagcta cgttgctaac 6060

gcaacatctc aagaagacag cggccaaagg gctcctccac tttgtgctag aaacgttaaa 6120

atacggcggc aacatagata ccgtcctgac ccaagccgta aagtacaatc atagaaaact 6180

tttagattat tttctgcgtc aactacctcg taaacatatt gaaaaacttt tgttgctggc 6240

cgtgcaggaa aaggcttcta aaaaaacatt gaacttactg ttgtcacatt taaactactc 6300

cgtgaaacgc atcaaaaaac taccgcgcta tgtgatagag tacgagtcca ccttggtgat 6360

aaagatttta ttaaaaaaaa gagtgaacct gatagatgcc atgttggaaa agatggtaag 6420

atatttttct gcgacgaaag tgaggacgat catggatgag ctttcgatta gtccggaaag 6480

agtcattaag atggctatac agaaaatgag aacggatatc gtaatccata cttcttatgt 6540

ttgggaggat gatctagaac gtcttactcg tcttaaaaat atggtataca ccataaagta 6600

cgaacatggg aaaaaaatgt taattaaagt catgcacggc atatacaaaa acttattata 6660

cggcgaaagg gaaaaagtca tgttttattt agccaagctc tatgttgctc aaaacgcggc 6720

cacccaattc agagacattt gtaaggactg ttacaaactg gatgtggcac ggtttaaacc 6780

gcggtttaag caactaatat tagactgttt agaaattatt actaaaaaat cttgctatag 6840

tatcctggaa atcttagaaa aacatattat ttccctgttt actatgaaag ttatgactga 6900

agaagaaaaa aacctatgtt tagaaatatt atataaagta attcattata aaacaataca 6960

atgttaaaat tcaatagata tccatcatta atattgatta tattttcgaa tattatcttc 7020

tatggtgcaa gataatcatc tagcgcgtga aacatgtcct cttctcttca ggaactttgt 7080

cgaaaaaagc tgcctgactg catacttcca gagttttttg acgactatgt attgcaactg 7140

ttaggactgc actggcaaga tcatggttcc cttcagcgta tcgagaagaa ccagatactt 7200

gttcaacagg aacccatcca tatcaatgaa gcactcaaag tagcagcatc ggaagggaac 7260

tatgaaatcg tagagctgtt gttgtcatgg gaggcagatc cccgctacgc cgtcgtagga 7320

gccctagaaa gcaaatacta tgacctggtt tacaaatact atgaccaagt taaagactgc 7380

catgatatct tgccgctgat tcaaaatccg gaaacattcg aaagatgtca tgagttaaac 7440

agcacctgtt cactgaaatg cttattcaag catgctgtga taaatgacat gctgccgatt 7500

cttcaaaaat atacagacta tctggatagg tgggagtatt gcagccagat gctgttcgaa 7560

ctggcatgta gtaaaaaaaa atatgagatg gttgtgtgga tagagggagt tctaggcgtc 7620

ggcaaagtta catctctttt caccattgcg attagcaaca gagacctaca gctgtattct 7680

ctgggctact caattatcct tgagaatttg tactcctgtg gacaggaccc caagttttta 7740

ctaaatcatt tcctgcgaga cgtttcaata aaagggcttc taccctttgt aatcaaaacc 7800

atagaatatg gtggaagcaa ggagatagcc ataactctgg ctaaaaaata tcagcataaa 7860

catattttga aatacttcga aacctgggaa agctaggttc agtatggtgt actcactatt 7920

gtagtgaatc gtatcctgta aattttgtaa aaaagcttaa acttttgacc acatcatatt 7980

gttttagaaa tctcaaacca gtgaacaaca gtcttatcat acattaaaat tccagtaaaa 8040

tttatatttt ttttggtaaa caaatgtttt ctcttcaaga catctgtcgg aaacatcttt 8100

ttcaacttcc tgacgctttt gatgaatata tattacaagc gctaggacta tactgggaaa 8160

aacacggatc tcttcaacga ataagaaagg acgctgtgtt tgtacagcga aacatcgtcc 8220

tttctaccaa tgaggccctg agaatcgcag cctcagaggg aaacgaaagg gtaataaaac 8280

ttctgttatc atgggaggga aattttcatt atgtgatcat aggagctcta gagggtgacc 8340

aatatgacct aattcataag tatgatagtc aaattaaaga ctaccacatg attttatcat 8400

tgatccaaaa tgcaaatacc tttgaaaagt gtcatcagtt atccaatagt aatatgtggt 8460

gtcttataca gaatgctata aaatataata tgctccctat tctccaaaaa cacagaaata 8520

ttctgacaca tgagggagag aatcaggaat tgtttgagat ggcatgtgag gaacagaaat 8580

atgacatagt tttatggata ggacaaaccc taatgttaaa tgagccggag tttatttttg 8640

atatcgcctt cgaacggata gatttttctt tattaacaat gggttatagc cttctttttg 8700

ataacaagat gagtagtata gacattcatg atgaagaaga tcttacttca ttaccaacag 8760

aacacctcga aaaagcagcc actaagggat gtttcttctt tatgctagaa actttaaaac 8820

atggtggaaa tgtaaatatg gcagtcttat ctaaagctgt tgagtataat catagaaaaa 8880

ttttagacca ttttattcgg cggcaaaaat gtttatcacg tgaagagatt gaaaacctat 8940

tattaaccgc cataaccaat tgtgcatcca taaaaacgtt aaacttactc ttgtcttacc 9000

taaactattc cgtaaaaaat atcattggaa aaatagtaca acatgtcata aaagatggtg 9060

attataccat catattactt ttaaaaaaaa agaaaataaa cctagtggaa cctgttttaa 9120

caggttttat agattattac tatagctatt gttttataaa acattttatc caagagtttg 9180

ctattcgtcc ggaaaaactg attaaaatgg ccgcgcgaaa aggtaaacta aatatgatta 9240

tcgaattcct taacgaaaaa tatgttcata aagatgatct tggaactata tttaaatatc 9300

tcaaaaccct agtatgtacc atgaaacata aaaaaggaaa agagacatta attgttctta 9360

ttcataaaat atatcaagat attcatctgg agactaaaga aaaatttaaa ttattaagat 9420

tttatgtcat gcatgatgca actatccaat ttctatctat gtgcaaagac tgttttaatt 9480

tagccggttt taaaccattt gttttagaat gtttggatat tgctattaaa aaaaattacc 9540

ctgatatgat acaatatata gaaattctat cgaaatctga gtaa 9584

<210> 4
<211> 21
<212> DNA
<213> Artificial Sequence

<220>
<223> Primer

<400> 4
tgctttcaag cctacaactc c 21

<210> 5
<211> 20
<212> DNA
<213> Artificial Sequence

<220>

<223>  Primer

<400>  5
attctcaggg cctcattggt                                                    20


<210>  6
<211>  21
<212>  DNA
<213>  Artificial Sequence

<220>
<223>  Probe

<400>  6
agcgatcctt tggctgccac c                                                  21


<210>  7
<211>  20
<212>  DNA
<213>  Artificial Sequence

<220>
<223>  Primer

<400>  7
tggcaagatc atggttccct                                                    20


<210>  8
<211>  20
<212>  DNA
<213>  Artificial Sequence

<220>
<223>  Primer

<400>  8
atctgcctcc catgacaaca                                                    20


<210>  9
<211>  27
<212>  DNA
<213>  Artificial Sequence

<220>
<223>  Probe

<400>  9
cccttccgat gctgctactt tgagtgc                                            27

## Description

### Technical field

The present invention refers to an attenuated African Swine Fever (ASF) virus and to its use as a vaccine containing it, for preventing ASF, as well as an in vitro method for the differential detection of an attenuated ASF virus and of the corresponding non-attenuated ASF virus.

Therefore, the present invention has utility in pharmaceutical, and especially veterinary fields.

In the description below, the references into brackets ([ ]) refer to the listing of references situated at the end of the text.

### Background of the Invention

African Swine Fever (ASF) is a viral disease that causes an often fatal hemorrhagic syndrome in its acute forms in domestic and wild suidae. Contagious in European swines, it is inapparent in wild African suidae (bushpigs and warthogs) and not transmissible to humans.

This disease has existed for at least a century in wild suidae from sub-Saharan Africa (bushpigs, warthogs and other hogs), but they do not develop any symptoms. Described for the first time in Kenya in 1921, ASF has spread in Africa from individual to individual and through soft tick bites, and has become endemic in sub-Saharan Africa.

His first forays outside Africa date back to the 1960s, in connection with the development of international trade. American outbreaks were eradicated fairly quickly like the European ones, except for the Iberian Peninsula where it was not until 1995 and for Sardinia where ASF has become enzootic since its introduction in 1978. In 2007, the European continent was again affected, with a first detection of outbreaks in pig farming in Georgia. In 2008, the disease progressively colonized the Eurasian continent (Armenia, Azerbaijan, Southern Russia), affecting both wild and domestic pigs. It continues to spread westwards, by spreading locally in small, poorly controlled farms. In 2014, the ASF returned to the European Union, first in Poland and the Baltic countries, where it has since become enzootic in wild boar, then in Moldova (2016), Romania and the Czech Republic (2017), Hungary and Belgium (2018) and in Germany (2020). Finally, since August 2018, the Asian continent has been largely affected: China, then Mongolia, Taiwan and Vietnam, the Philippines (in 2019), and India in 2020. The risk of diffusion has become global.

Depending on the virulence of the virus strain and the susceptibility of the pig host, ASF can be presented with different virulence levels of the disease: the acute form, the sub-acute form and the chronic form.

The symptoms and lesions are similar to those described for classical swine fever, namely hyperthermia, haematological disorders, skin redness, anorexia, lethargy, coordination disorders, vomiting and diarrhoea.

Death occurs in 4 to 13 days with a rate of 100% in the acute form, in 30 to 40 days with less mortality in the sub-acute form. The disease can progress for several months in the chronic form.

The virus responsible for ASF, the only member of viral family *Asfarviridae,* is a very complex and large enveloped double-stranded DNA virus. It infects the cells of the monocyte-macrophage lineage and diverts the immune response to its advantage. Its genome has only been fully sequenced in recent years and not all the genes involved in virulence or protection have yet been identified, delaying the development of a reliable and effective vaccine.

ASF is classified as a first category health hazard in France with respect of the hierarchy of health hazards associated with animal health currently in force. In animal health, first category of hazards concerns serious public health problems or major risks to the environment or French production capacities. They require, in the general interest, compulsory prevention, monitoring or control measures. This disease causes major economic losses due to its high mortality rate and the trade restrictions imposed on affected countries.

The diagnosis of this disease is complex, and to date only laboratory tests (virological and/or serological) can provide a diagnosis and differentiate ASF from classical swine fever. First-line diagnosis can be made, for rapid detection (within 24 hours), by ELISA for serology and PCR for virology, using fully validated methods, some of them being available as commercial kits.

Finally, to date, there is no treatment or vaccine to control the disease, and the only means of controlling this disease remains quarantine and slaughter. The challenge within the European Union is to have a vaccine that could be delivered orally to wild boar, for example by bait, as already done for classical swine fever, or to pigs in small family farms. The challenge at the global level is to have a vaccine for breeding pigs, especially for Asia, which is the world's leading producer.

Attempts to vaccinate animals using infected cell extracts, supernatants of infected pig peripheral blood leukocytes, purified and inactivated virions, infected glutaraldehyde-fixed macrophages, or detergent-treated infected alveolar macrophages failed to induce protective immunity. Homologous protective immunity does develop in pigs surviving viral infection. Pigs surviving acute infection with moderately virulent or attenuated variants of ASFV develop long-term resistance to homologous, but rarely to heterologous, virus challenge. Pigs immunized with live attenuated ASF viruses containing engineered deletions of specific ASFV virulence-associated genes were protected when challenged with homologous parental virus. Specifically, individual deletion of UK (DP69R), 23-NL (DP71L), TK (A240L) or 9GL (B119L) genes from the genomes of pathogenic ASF viruses (Malawi LiI-20/1, Pretoriuskop/96/4, and E70) markedly attenuated the virus in swine and the animals immunized with these attenuated viruses were protected against challenge with homologous virus.

Variations in genome size and restriction fragment patterns are observed among different ASFV isolates and this diversity resides in the terminal genomic regions. These variable regions comprise the left 35-kb and the right 15-kb ends of the ASFV genome and contain at least five multigene families (MGFs): MGF 100, MGF 110, MGF 300, MGF 360, and MGF 505. These genes are grouped in these MGFs as they share both sequence and structural identity. The functions of these genes are not fully understood, but they have been correlated with macrophage host range, modulation of the innate host immune response and virulence.

Recently, a recombinant ASFV-G (African Swine Fever Virus-Georgia 2007 isolate) mutant, the "ASFV-G-ΔMGF", comprising a deletion of 7,559 nucleotides (between nucleotide positions 27,928-35,487) resulting in a deletion of MGF 360 genes 12L, 13L, and 14L along with the MGF505 1R, 2R and 3R, has been created (O'Donnell et al.: "African Swine Fever Virus Georgia Isolate Harboring Deletions of MGF360 and MGF505 Genes Is Attenuated in Swine and Confers Protection against Challenge with Virulent Parental Virus", J Virol. 2015 Jun;89(11):6048-56 **([1])** ; US20160130562 **([2])).** However, if this mutant virus protected clinically the pigs against a lethal challenge with the ASF Georgia strain, a significant part of the immunized pigs (4 out of 10) were not virologicaly protected as they displayed a viremia after the challenge.

Thus, a need exists of alternative attenuated vaccine. The present invention fulfills this and other needs.

### Description of the invention

As a result of significant work, the Applicant has developed a new attenuated African Swine Fever virus, internal reference PPA/dp/FR-22/2018/180172-2, further named ASFV-989, which surprisingly shows superior vaccine efficacy compared to existing attenuated virus, and especially to ASFV-G-ΔMGF, by completely preventing viremia in challenged individuals.

Surprisingly, the inventors found that the attenuated ASFV-989 of the invention shows similar replication rate to the unattenuated Georgia 2007/1 when cultivated on porcine alveolar macrophages.

The inventors have shown a cellular response specific to the ASF virus in animals inoculated with the attenuated virus of the invention (either by intramuscular or oronasal route), a seroconversion and a viraemia 100 to 1000 times lower in animals inoculated with the attenuated virus of the invention ASFV-989 than in animals inoculated with the Georgia 2007/1 strain. Interestingly, after challenge with the Georgia strain, the animals did not display any ASF associated symptoms and no viremia was detected for this strain in animals previously immunized with the attenuated virus of the invention. In addition, no genome of the Georgia strain was detected in the tissues of animals previously immunized with the attenuated virus of the invention via intramuscular route.

Furthermore, the Applicant has developed a molecular method for the differential detection, in a sample, of the attenuated ASFV of the invention, and of the corresponding non-attenuated ASFV.

Accordingly, in a first aspect, the present invention provides an attenuated African Swine Fever (ASF-989) virus, wherein :
- genes MGF 360-12L, 360-13L, 360-14L, 505-2R, 505-3R are deleted or are interrupted or mutated such that the genes are not transcribed and/or translated,
- ORF of ASFV_G_ACD_00520 is deleted or is interrupted or mutated such that it is not transcribed and/or translated, and
- genes MGF 505-1R et 505-4R are truncated, compared to the genome of the corresponding unattenuated virus.

The "genome of the corresponding unattenuated virus" refers herein to the genome from the corresponding virus that does not contain the modifications listed above. It may be a virulent ASFV virus strain selected among the lineage of Georgia 2007/1, e.g. Pig/HLJ/2018 (Xuexia Wen et al. (2019) Genome sequences derived from pig and dried blood pig feed samples provide important insights into the transmission of African swine fever virus in China in 2018, Emerging Microbes & Infections, 8:1, 303-306 **([3])**), ASFV-SY18 (Xuexia Wen et al. **([3])),** China/2018/AnhuiXCGQ (Bao, J et al. Genome comparison of African swine fever virus China/2018/AnhuiXCGQ strain and related European p72 Genotype II strains. Transbound Emerg Dis. 2019; 66: 1167- 1176 **([4])),** ASFV/POL/2015/Podlaskie (Mazur-Panasiuk N. et al. The first complete genomic sequences of African swine fever virus isolated in Poland. Sci Rep 9, 4556 (2019) **([5])),** Odintsovo 2/2014, Kashino 04/13, ASFV-Belgium2018/1 (Forth JH et al. Comparative Analysis of Whole-Genome Sequence of African Swine Fever Virus Belgium 2018/1. Emerg Infect Dis. 2019 Jun;25(6):1249-1252 **([6])**) or ASFV/Kyiv/2016/131 (Kovalenko, G. et al. Complete Genome Sequence of a Virulent African Swine Fever Virus from a Domestic Pig in Ukraine. Microbiology resource announcements 2019, 8, e00883-00819 **([7])),** a strain of ASF virus of genotype II or a genetically close ASF virus strains.

According to the invention, the deletions may be made by any means known by the skilled person. The deletion may be a mutation in which at least a part of each gene or ORF is ablated. Any number of nucleotides can be deleted, from a single base to the entire gene or ORF, as long as the genes or ORF are not transcribed and/or translated. Preferably, the entire genes and ORF are deleted, i.e.:
- genes MGF 360-12L, 360-13L, 360-14L, 505-2R, 505-3R are entirely deleted, and
- ORF of ASFV_G_ACD_00520 is entirely deleted.

According to the invention, the interruptions may be made by any means known by the skilled person. It may be for example by deleting or otherwise modifying the ATG start codon of a gene, or by modifying a translational start site, or a frame shift; or introduction of one or more stop codons in the open reading frame of the gene.

According to the invention, the mutations may be made by any means known by the skilled person, for example, by a change in the structure of the gene, affecting one to several nucleotides. It may be a substitution mutation, i.e. the replacement of at least one nucleotide by another, or an insertion mutation, i.e. the addition of one or more nucleotides, or a deletion mutation, i.e. the loss of one or more nucleotides, or a mutation by inversion, i.e. the permutation of 2 neighboring deoxyribonucleotides. In any case, the mutation, in order to be expressed, is a substitution by one or more nucleotides giving a codon encoding an amino acid different from that given by the initial nucleotide, otherwise the mutation is said to be silent. In any case, the mutation may cause the genes to not be transcribed and/or translated.

According to the invention, the phrase "not transcribed and/or translated" means that there is no transcription and/or no translation at all of the corresponding genes or ORF.

According to the invention, the truncation may be a deletion of any part of the genes, may be made by any means known by the skilled person, for example by inserting in at least one gene a point-nonsense mutation, i.e. a point mutation in the DNA sequence that results in a premature stop codon, and in a truncated, incomplete, and optionally nonfunctional protein product. Preferably, the start codon ATG at the beginning of MGF 505-1R gene and the stop codon at the end of MGF 505-4R gene are maintained, and so are not part of the truncated portion. In this case, the two parts of the MGF 505-1R and 505-4R genes form 2 shorter proteins 1R and 4R.

In the embodiment wherein:
- genes MGF 360-12L, 360-13L, 360-14L, 505-2R, 505-3R are deleted,
- ORF of ASFV_G_ACD_00520 is deleted, and
- genes MGF 505-1R et 505-4R are truncated, wherein the start codon ATG at the beginning of MGF 505-1R gene and the stop codon at the end of MGF 505-4R gene are maintained,
- 7458 nucleotides are deleted compared to the genome of Georgia 2007/1 strain (FR682468.2). In this embodiment, the nucleotide sequence of MGF genes is SEQ ID NO : 1. The 7458 nucleotides sequences of the deleted region is shown as SEQ ID NO : 2, and the MGF genes nucleotide sequence of Georgia strain 2007/1 is SEQ ID NO : 3.

The attenuated ASF virus of the invention may be obtained by directed mutagenesis, for example by homologous recombination or CRISPR-Cas9. Alternatively, the attenuated ASF virus of the invention may be obtained by thermal attenuation of a virulent ASFV virus strain as described below. Therefore, another object of the invention is an in-vitro method for obtaining an attenuated ASF virus of the invention, which comprises the steps of:
- Deleting, interrupting or mutating genes MGF 360-12L, 360-13L, 360-14L, 505-2R, 505-3R so that the genes are not transcribed and/or translated,
- Deleting, interrupting or mutating ORF of ASFV_G_ACD_00520 so it is not transcribed and/or translated,
- Truncating genes MGF 505-1R et 505-4R,
compared to the genome of the corresponding unattenuated virus.

Obviously, any classical step implemented in directed mutagenesis processes can be carried out as part of the process of the invention, for example PCR, cloning, sequence analysis and amplification. To this purposes, it may be a process as described by O'Donnell V et al. **(([1]))** or Rathakrishnan A et al. (Production of Recombinant African Swine Fever Viruses: Speeding Up the Process. Viruses. 2020 Jun 5;12(6):615 **([8])).** Preferably, as already described above :
- genes MGF 360-12L, 360-13L, 360-14L, 505-2R, 505-3R are deleted, and
- ORF of ASFV_G_ACD_00520 is deleted.

Alternatively:
- genes MGF 360-12L, 360-13L, 360-14L, 505-2R, 505-3R are interrupted such that the genes are not transcribed and/or translated,
- ORF of ASFV_G_ACD_00520 is interrupted such that it is not transcribed and/or translated.

It is yet another object of this invention to provide an in vitro method for obtaining an attenuated ASF virus of the invention, which comprises at least one step of thermal-attenuation of a virulent ASFV virus strain selected among Georgia 2007/1, Pig/HLJ/2018, a strain of ASF virus of genotype II or a genetically close ASF virus strain, and amplifying and selecting said attenuated ASF virus.

Advantageously, the thermal attenuation may be realised in one or more step(s), and is thus a continued or discontinued process. The virulent ASFV virus strain may be subjected to a temperature ranging between 55 to 65°C, depending on the duration of each step of thermal attenuation. The skilled person will be able to vary the temperature according to the duration of the thermal inactivation in view of his general knowledge, and by verifying the obtaining of the virus of the invention by sequence analysis.

The steps of amplification and selection may be realised by method commonly used by the skilled person in this technical field, for example by inoculation of Specific-Pathogen-Free pigs (known as highly susceptible to infection) for amplifying the attenuated virus, and sequence analysis for selecting it.

Whether the virus of the invention is obtained by directed mutagenesis or thermal inactivation, as described above, it does not contain any reporter gene. This is an advantage over the attenuated virus of the prior art, which contain such a reporter gene, especially GUS or p72-cherry that confer a GMO status to the vaccine or pharmaceutical composition.

In another aspect, the present invention provides a vaccine comprising an attenuated ASF virus of the invention.

The term "vaccine" as used herein refers to a preparation which, when administered to a subject, induces or stimulates a protective immune response in an animal to which the vaccine has been delivered. A vaccine can render an organism immune to a particular disease, in the present case ASF. The vaccine of the present invention thus induces an immune response in a subject, which is protective against subsequent ASF virus challenge. Therefore, the vaccine may be useful in preventing African Swine Fever in vaccinated animals. The term "preventing" is intended to refer to averting, delaying, impeding or hindering the contraction of ASF. The vaccine may, for example, prevent or reduce the likelihood of an infectious ASFV entering a cell, the occurrence of clinical symptoms of the disease and mortality. In an embodiment, the vaccine may comprise different strains of said attenuated ASF virus. Such a vaccine may be capable of inducing a cross-protective immune response against a plurality of ASF virus strains.

The vaccine of the invention may be in the form of a pharmaceutical composition.

The vaccine or pharmaceutical composition of the invention may comprise the attenuated ASF virus of the invention and optionally one or more adjuvants, excipients, carriers and diluents commonly used in the field of the invention.

Carriers may include stabilizers, preservatives and buffers. Suitable stabilizers are, for example SPGA (sucrose, phosphate, glutamate, and human. albumin), carbohydrates (such as sorbitol, mannitol, starch, sucrose, dextran, glutamate or glucose), proteins (such as dried milk serum, albumin or casein) or degradation products thereof. Suitable buffers are for example alkali metal phosphates. Suitable preservatives are thimerosal, merthiolate and gentamicin. Diluents include water, aqueous buffer (such as buffered saline), alcohols and polyols (such as glycerol).

Preferably, the composition of the invention as medicinal product could further comprises immunity adjuvants; these adjuvants may be of any type known to those skilled in the art provided that, when they are mixed with an efficient quantity of live attenuated ASFV according to the present invention, they allow the antigenic power of the composition to be increased and promote a greater immune reaction.

A vaccine or pharmaceutical composition according to the invention can be prepared by conventional methods such as those commonly used for the commercially available live attenuated virus vaccines. Briefly, a susceptible substrate is inoculated with the ASF virus of the invention and propagated until the virus has replicated to a desired titer after which virus-containing material is harvested. Subsequently, the harvested material is formulated into a pharmaceutical preparation with immunizing properties.

In another aspect, the present invention provides an attenuated ASF virus according to the invention, or a vaccine according to the invention, for use in preventing African Swine Fever in a subject.

Advantageously, the attenuated ASF virus or a vaccine of the invention may induce in the subject a protective immune response against the virulent ASFV virus strain Georgia 2007/1 or against other genetically close ASF virus strains.

Preferably, the vaccine or the attenuated ASF virus may be administered as a single dose, corresponding to a prime regimen. Alternatively, the vaccine of the invention may be administrated in several doses.

The vaccine of the invention may be administered by any convenient route, especially by a route selected among oronasal route and parenteral route, especially intra-muscular route. In one embodiment, oral administration comprises adding the vaccine to animal feed or drinking water. In another embodiment, the vaccine may be added to a bait for a wild animal, for example a bait suitable for wild boar, wild pigs, bushpigs or warthogs. Preferably, the administration is a prime administration by oronasal route.

The invention further provides a method for preventing African Swine Fever in a subject which comprises the step of administering an effective amount of an attenuated ASF virus to a subject in need thereof.

In another aspect, the present invention provides an in vitro method for the differential detection, in a sample, of an attenuated ASF virus of the invention, and of the corresponding non-attenuated ASF virus, which comprises carrying out differential amplification of the nucleic acids strands by a molecular method such as PCR with sets of primers / probes specific for each strain, wherein primers / probe of sequences:
Del_Fow TGCTTTCAAGCCTACAACTCC (SEQ ID NO: 4),
Del_Rev ATTCTCAGGGCCTCATTGGT (SEQ ID NO: 5) and
Del_Probe AGCGATCCTTTGGCTGCCACC (SEQ ID NO: 6)
allows the specific amplification of the attenuated ASF virus, whereas the primers / probe of sequences :
505_3R_L1 TGGCAAGATCATGGTTCCCT (SEQ ID NO: 7),
505_3R_R1 ATCTGCCTCCCATGACAACA (SEQ ID NO: 8) and
505_3R_P1 CCCTTCCGATGCTGCTACTTTGAGTGC (SEQ ID NO: 9)
allows the specific amplification of the corresponding non-attenuated ASF virus.

The differential amplification may be realized under experimental conditions commonly used in this technical field.

Advantageously, the method of the invention allows to distinguish animals infected with the non-attenuated ASF virus from animals vaccinated with the attenuated ASF virus of the invention.

The sample of the animal used in this method may be any sample in which the virus can be detected, for example a blood sample or tissues samples such as spleen, tonsil or lymph nodes.

A further object of the invention is to provide genetic markers which can distinguish between vaccinated animals and animals infected with non-attenuated ASF virus, consisting of at least one sequence among SEQ ID NO: 4, SEQ ID NO: 5 and SEQ ID NO: 6.

This invention is further illustrated by the following examples with regard to the annexed drawings that should not be construed as limiting.

### Brief description of the figures

- Figure 1: represents the comparison of the Georgia 2007/1 and ASFV-989 genomes at the site of the deletion.
- Figure 2: represents the viral replication of Georgia 2007/1 and ASFV-989 strains in Porcine alveolar macrophages (Titer in Log10 HAD50/ml, time in Days post-inoculation).
- Figure 3: represents the position of the primers and probes for the PCR 505 (L1, P1 and R1) detecting the Georgia 2007/1 strain and the PCR 989 (Del_Fow, Del_probe and Del_Rev) detecting the ASFV-989.
- Figure 4: represents the evolution of rectal temperature (°C) after IM immunization with Georgia or ASFV-989 strains (time in Days post-immunization). Control: data from group A1; 989 IM: data from groups C & D; Geo IM: data from group E.
- Figure 5: represents pigs survival (%) after IM inoculation (time in Days post-inoculation) with the Georgia or ASFV-989 strains. Control: data from group A1; 989 IM: data from groups C & D; Geo IM: data from group E.
- Figure 6 represents the evolution of growth performances after IM inoculation with Georgia or ASFV-989 strains. Average daily weight gain (Kg/D) post-inoculation. Control: data from groups A1 & A2; 989 IM: data from groups C & D; Geo IM: data from group E.
- Figure 7 represents the evolution of rectal temperature (°C) after ON immunization with Georgia or ASFV-989 strains, in days. Control: data from group A1; 989 ON: data from groups B, H & I; Geo ON: data from group F.
- Figure 8 represents pigs survival (%) after ON inoculation with the Georgia or ASFV-989 strains, in days. Control: data from group A1; 989 ON: data from groups B, H & I; Geo ON: data from group F.
- Figure 9 represents the evolution of growth performances (Average daily weight gain (Kg/D)) after ON inoculation with Georgia or ASFV-989 strains. Control: data from group A1 & A2; 989 ON: data from groups B, H & I; Geo ON: data from group F.
- Figure 10 represents evolution (days post-inoculation) of ASFV viremia (Log10 eq HAD50/ml) after inoculation with Georgia or ASFV-989 strains. Geo IM: data from groups E & F; Geo ON: data from groups F & J; 989 IM: data from groups C & D; 989 ON: data from groups B, H & I.
- Figure 11 represents the evolution (days) of ASFV viremia (PCR 989, Log10 eq HAD50/ml) after inoculation with ASFV-989 strain. 989 IM: data from groups C & D; 989 ON: data from groups B, H & I.
- Figure 12 represents the antibody response (detected by ELISA, Competition %) after inoculation with Georgia or ASFV-989 strains (Days post-inoculation). 989 IM: data from group C; 989 ON: data from group B; Geo IM: data from group E; Geo ON: data from group F.
- Figure 13 represents the specific cell mediated response (Number of IFNg secreting cells / 10⁶ PBMC) after inoculation with ASFV-989 strain (Days post-inoculation). 989 IM: data from groups C & D; 989 ON: data from groups B, H & I.
- Figure 14 represents the evolution of rectal temperature (°C) after IM challenge with Georgia ASFV strain (days post-challenge). Control: data from group A2; 989 IM / Geo IM: data from group D; 989 ON / Geo IM: data from group I; Geo IM: data from group G.
- Figure 15 represents the evolution of rectal temperature (°C) after ON challenge with Georgia ASFV strain (days post-challenge). Control: data from group A2; 989 ON / Geo ON: data from group H; Geo ON: data from group J.
- Figure 16 represents the evolution of growth performances (Average daily weight gain (Kg/j)) after challenge with Georgia ASFV strain (days post-challenge). Control: data from group A1 & A2; 989 IM / Geo IM: data from group D; 989 ON / Geo ON: data from group H; 989 ON / Geo IM: data from group I; Geo ON: data from group J.

### Examples

### Example 1: Preparation and in vivo characterization of ASFV-989 strain

### 1. Material and method

### 1.1 Viruses and cells

Georgia 2007/1 ASFV strain, initially isolated in 2007 from a domestic pig originating in Georgia, was kindly provided by Dr. Linda Dixon (OIE reference laboratory, Pirbright Institute, UK). The PPA/dp/FR-22/2018/180172-2 ASFV strain, thereafter named ASFV-989 (or "989"), was isolated from a blood sample collected on a specific pathogen free (SPF) pig that was inoculated with the thermo-attenuated ASFV Georgia 2007/1 strain. Georgia 2007/1 and ASFV-989 strains were cultured on porcine alveolar macrophages (PAM) for 3 passages and 1 passage respectively before being inoculated in pigs. Viruses were diluted in RPMI medium to adjust the inoculation dose to 10³ hemadsorbing dose 50% (HAD₅₀) per pig for intramuscular (IM) inoculation and 10⁴ HAD₅₀ per pig for oronasal (ON) inoculation. Virus titration was performed by hemadsorption assay (Carrascosa, A.L. et al. Methods for growing and titrating African swine fever virus: field and laboratory samples. Curr Protoc Cell Biol, 2011. Chapter 26: p. Unit 26 14 **([9])).**

### 1.2 Full genome sequencing

DNA was extracted from the ASFV-989 strain with the High Pure PCR Template Preparation Kit (Roche Diagnostics, Meylan, France). For library preparation, Ion Xpress Plus Fragment Library Kit (Thermo Fischer Scientific, Frederick, Maryland, USA) was used and DNA fragments between 250 bp and 290 bp were size-selected with Ion Xpress Barcode Adapters 1-96 kit (Thermo Fischer Scientific, Frederick, Maryland, USA). For the DNA purification steps, magnetic beads from Agencourt AMPure XP Kit (Beckman Coulter, Villepinte, France) were used. All samples were sequenced with Proton Ion Torrent technology (Thermofischer Scientific, Frederick, Maryland, USA). The reads were cleaned with the Trimmomatic (Bolger, A.M., M. Lohse, and B. Usadel, Trimmomatic: a flexible trimmer for Illumina sequence data. Bioinformatics, 2014. 30(15): p. 2114-20 **([10]))** 0.36 software (ILLUMINACLIP: oligos.fasta: 2:30:5:1: true; LEADING: 3; TRAILING: 3; MAXINFO: 40:0.2; MINLEN: 36). Then a bwa (Li, H. and R. Durbin, Fast and accurate short read alignment with Burrows-Wheeler transform. Bioinformatics, 2009. 25(14): p. 1754-60 **([11]))** (version 2.2.5) alignment was performed with cleaned reads versus NCBI reference FR682468.2. The consensus sequence was created with samtools (Li, H., et al., The Sequence Alignment/Map format and SAMtools. Bioinformatics, 2009. 25(16): p. 2078-9 **([12]))** (version 1.8) and seqtk (version 1.2) (https://github.com/lh3/segtk)

### 1.2 In vivo study

Fifty Specific Pathogen Free (SPF) Large White pigs, 6 weeks-old, were used in the two trials described in Table 1.

**Table 1: experimental design for the in vivo study**

| **Group number** | **Group Name** | **Nb of pigs** | **Trial #** | **Inoculation (D0)** | **Challenge (D28)** | **Necropsy** |
|---|---|---|---|---|---|---|
| **A1** | **Control** | 3 | 1 | / | / | D100 |
| **B** | **989 ON LT** | 5 | 1 | ASFV-989 ON | / | D100 |
| **C** | **989 IM LT** | 5 | 1 | ASFV-989 IM | / | D100 |
| **D** | **989 IM / Geo IM** | 6 | 1 | ASFV-989 IM | Georgia IM | D68 |
| **E** | **Geo IM D0** | 3 | 1 | Georgia IM | | D6 |
| **F** | **Geo ON D0** | 4 | 1 | Georgia ON | | D6 |
| **G** | **Geo IM D28** | 3 | 1 | / | Georgia IM | D35 |
| **A2** | **Control** | 5 | 2 | / | / | / |
| **H** | **989 ON / Geo ON** | 6 | 2 | ASFV-989 ON | Georgia ON | D68 |
| **I** | **989 ON / Geo IM** | 6 | 2 | ASFV-989 ON | Georgia IM | D68 |
| **J** | **Geo ON D28** | 4 | 2 | | Georgia ON | D35 |

| | | | | | | |
|---|---|---|---|---|---|---|
| ON: oronasal; IM: intramuscular; LT: long term; Inoculum: 989 ON / Georgia ON: 10⁴ HAD50 / pig - 989 IM / Georgia IM: 10³ HAD50 / pig | | | | | | |

Groups of pigs were inoculated with ASFV-989 or Georgia strains at D0 either by intramuscular (IM) or oronasal (ON) route. Among the groups inoculated with the ASFV-989 strain (immunized), two were left unchallenged (989 ON and 989 IM long term: LT) whereas the others groups were challenged with the Georgia strain 28 days after the ASFV-989 inoculation. Control pigs (non-immunized, non-challenged) were also included in each trial.

Pigs were identified individually and each group of pig housed in separated rooms in the air-filtered biosafety level 3 animal facilities at Anses-Ploufragan. All pigs were monitored daily for rectal temperature and clinical signs of ASFV infection, and they were weighed once per week. Blood samples were collected before inoculation or challenge, then twice a week during the first 2 weeks after inoculation / challenge and finally once a week during the remaining of the follow-up. Blood was collected in tubes with lithium heparin for ELISPOT IFNg and virus isolation, EDTA for ASFV genome detection, and with dry tubes to obtain serum for antibody detection. At the end of the experiment, or at earlier stages for animal welfare reasons, pigs were sacrificed by anesthetic overdose and then exsanguinated.

### Ethics statement

Animal experiments were authorized by the French Ministry for Research (project N° 2019030418445731) and approved by the national ethics committee (authorization N° 19-018#19585).

### Virological and immunological assays

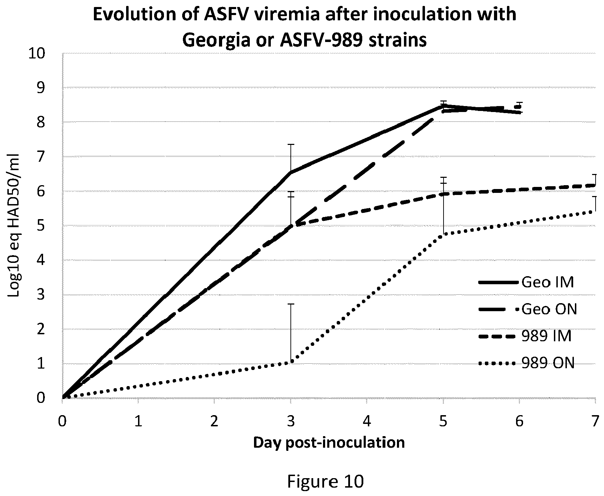

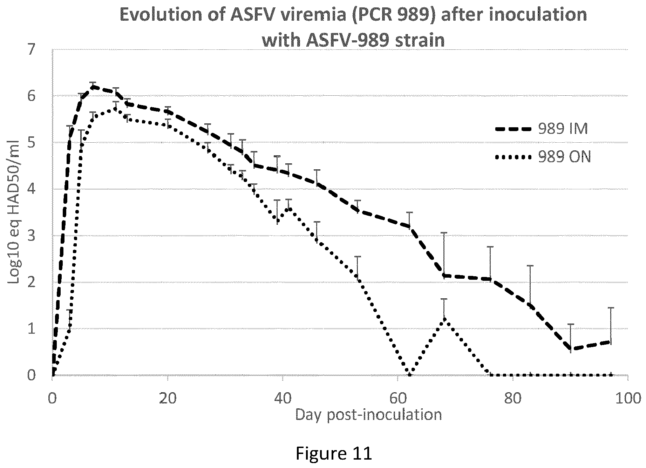

Real-time PCR: assessment of the ASFV viremia during the first week after inoculation (Figure 10) was performed by real-time PCR as previously described (Tignon, M., et al., Development and inter-laboratory validation study of an improved new real-time PCR assay with internal control for detection and laboratory diagnosis of African swine fever virus. J Virol Methods, 2011. 178(1-2): p. 161-70 **([13]))** using DNA extracted from 100µL of EDTA blood samples using the NucleoSpin^{®} 8 Virus (Macherey-Nagel). Pig Beta-actin was used as internal control for DNA extraction from pig samples. The long term follow-up of the ASFV-989 viremia (Figure 11) and the specific detection of ASFV-989 or Georgia genome in immunized then challenged pigs (Tables 2, 3 and 4) were respectively performed with PCR 989 and PCR 505 according to the method described in results part.

ELISA: Antibodies against protein P32 of the ASFV were measured in serum using a competition ELISA kit according the manufacturer's instructions (ID Screen ASFV competition, IDVET, France).

ELISPOT: ASFV-specific IFNg-secreting cells (IFNg-SCs) were quantified as previously described (King, K., et al., Protection of European domestic pigs from virulent African isolates of African swine fever virus by experimental immunisation. Vaccine, 2011. 29(28): p. 4593-600 **([14])),** using 16 h ASFV stimulation of 4x10⁵ PBMCs with a multiplicity of infection of 0.2 for the Georgia strain. The number of spots per well was counted using an ImmunoSpot S6 UV Analyzer (CTL, Shaker Heights, OH, USA).

### 2. Results

### 2.1 Genome sequence and in vitro characterization

### + Full genome sequence of the ASFV-989 strain

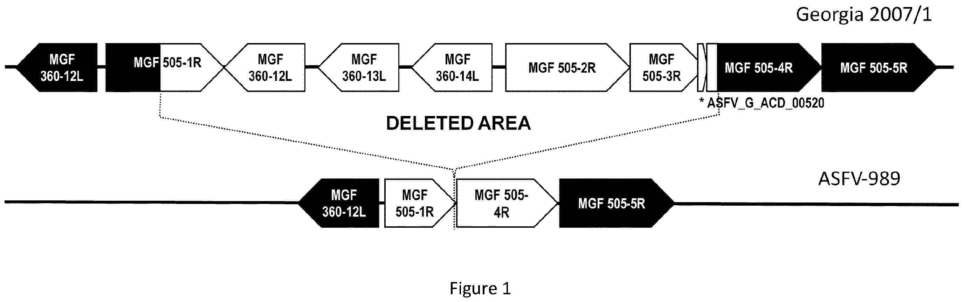

The ASFV-989 strain was isolated from a blood sample collected on a SPF pig (pig number 989) inoculated with the thermo-attenuated ASFV Georgia strain. The ASFV-989 strain was further amplified once on PAM and submitted to full genome sequencing. Comparison of ASFV-989 and Georgia full genome sequences (FR682468.2) revealed for the ASFV-989 strain a deletion of 7458 nucleotides (between nucleotide positions 29439 and 36898) corresponding to the partial deletion of MGF 505-1R et 505-4R and the complete deletion of MGF 360-12L, 360-13L, 360-14L, 505-2R, 505-3R and ASFV_G_ACD_00520. Figure 1

### + In vitro replication of the ASFV-989 and Georgia strains

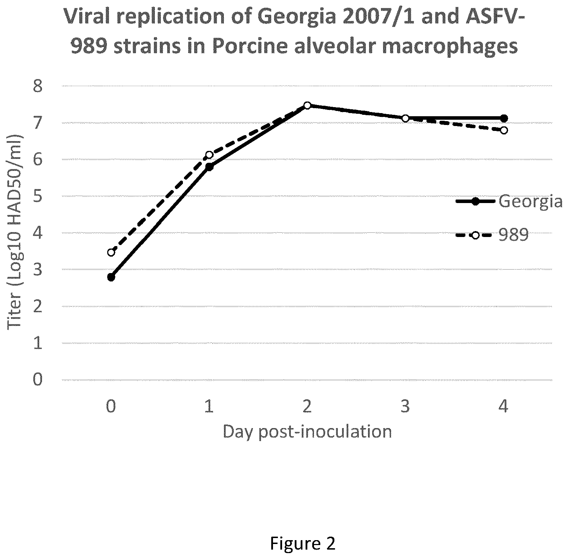

To characterize the *in vitro* replication of the ASFV-989 strain in comparison to its parental strain Georgia, Porcine Alveolar Macrophages (PAM) were infected with both strains and the replication kinetics were followed during 4 days. As depicted on Figure 2, the replication kinetics were similar for the ASFV-989 and the Georgia strains with an increase of the viral titer from D0 to D2 post-inoculation followed by a plateau from D2 to D4.

### + Design of specific PCR systems to detect the ASFV-989 and Georgia strains

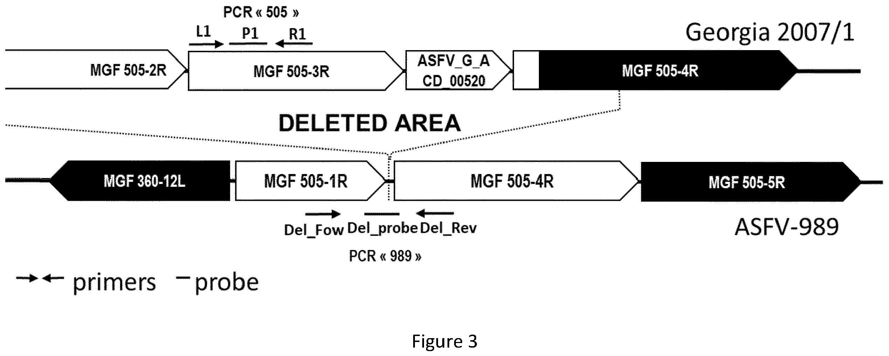

In order to have a PCR tool able to detect specifically the ASFV-989 or the Georgia strains in biological samples, we designed a PCR system for each of these strains. PCR 505 is targeting the MGF 505-3R gene of Georgia strain with primer and probe sequences as follow 505_3R_L1 TGGCAAGATCATGGTTCCCT (SEQ ID NO: 7), 505_3R_R1 ATCTGCCTCCCATGACAACA (SEQ ID NO: 8) and 505_3R_P1 CCCTTCCGATGCTGCTACTTTGAGTGC (SEQ ID NO: 9). PCR 989 is targeting the chimeric gene of the 989 strain resulting from the fusion of the proximal part of MGF 505-1R and the distal part of MGF 505-4R (Figure 3). The primers and probe sequences for PCR 989 are as follow: Del_Fow TGCTTTCAAGCCTACAACTCC (SEQ ID NO: 4), Del_Rev ATTCTCAGGGCCTCATTGGT (SEQ ID NO: 5) and Del_Probe AGCGATCCTTTGGCTGCCACC (SEQ ID NO: 6).

Firstly, we confirmed that the PCR 505 is detecting the Georgia strain but not the ASFV-989 strain and that the PCR 989 is detecting the ASFV-989 strain, but not the Georgia. Using a standard range of ASFV-989 or Georgia strains, we determined the PCR efficacy, linearity and the limit of quantification for each PCR. Both PCR presented an efficacy higher than 90%, a very good linearity (R² higher than 0.99 for five dilutions) with a limit of quantification of 10E3.8 equivalent HAD₅₀/ml.

### 2.2 In vivo characterization of the virulence and immune response to ASFV-989 strain inoculation, in comparison to Georgia strain

To characterize the virulence level and the immune response induced in pigs inoculated with the ASFV-989 strain, we conducted two *in vivo* trials during which we inoculated pigs either with the ASFV-989 or Georgia strains, by intramuscular (IM) or oronasal (ON) route. (Table 1)

In a first step we compared the clinical, virological and immunological parameters for pigs inoculated by the ON or IM route with the ASFV-989 strain to pigs receiving the virulent Georgia strain by the same routes. To increase the number of pigs per condition, the data from different experimental groups have been compiled. The experimental groups corresponding to each condition are mentioned for each figure.

### + Clinical and zootechnical data

### - Intramuscular inoculation :

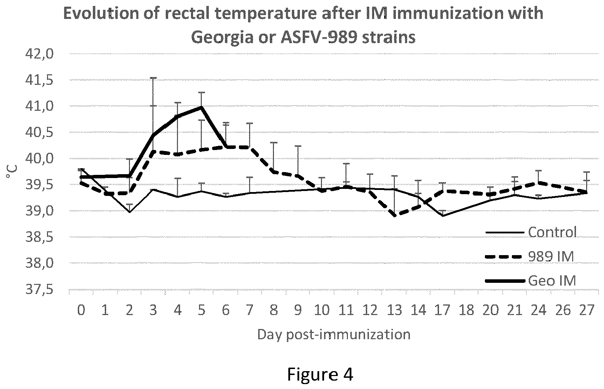

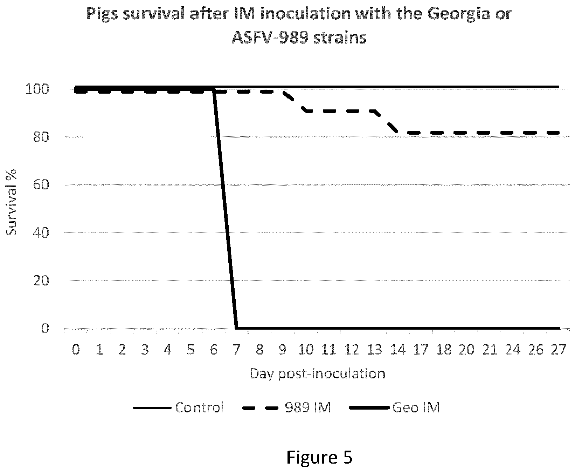

After IM inoculation of the Georgia strain, the infected pigs (Geo IM: group E) displayed the first hyperthermia at D3 post-inoculation (PI), then they showed a peak of hyperthermia 2 days later (41.0±0.3°C at D5 PI, Figure 4) and developed the typical ASF related symptoms. They have to be euthanatized at D6 PI (Figure 5).

The pigs inoculated intramuscularly with the ASFV-989 strain (989 IM: groups C and D) also showed the first hyperthermia at D3 PI, but developed lower hyperthermia (40.2±0.6°C at D5 PI, Figure 4) and limited symptoms.

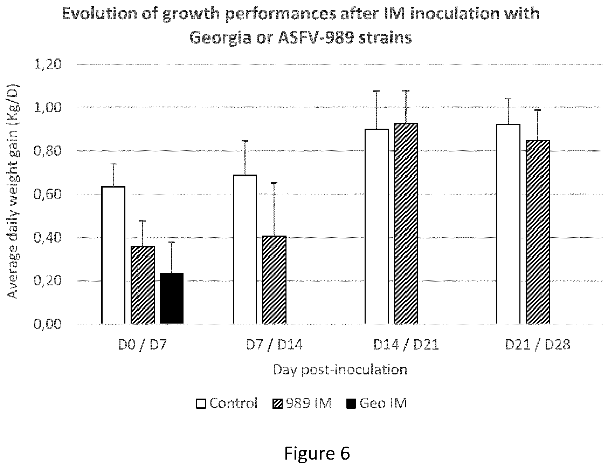

Among the 11 pigs inoculated IM with the ASFV-989 strain, only two animals developed ASF related symptoms during the 2 first weeks after ASFV-989 inoculation and had to be euthanatized (Figure 5). Increase in rectal temperature in the IM 989 inoculated pigs was accompanied with a decrease in growth performances during the 2 weeks after inoculation (Figure 6): average daily weight gain -ADWG- between D0/D7 PI: 0.36±0.12 kg/D for 989 IM versus 0.64±0.10 kg/D for control and ADWG between D7/D14 PI: 0.41±0.24 kg/D for 989 IM versus 0.69±0.16 kg/D for control.

### - Oronasal inoculation:

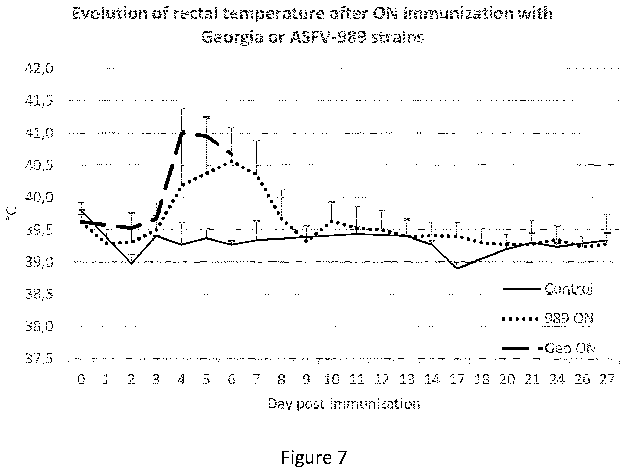

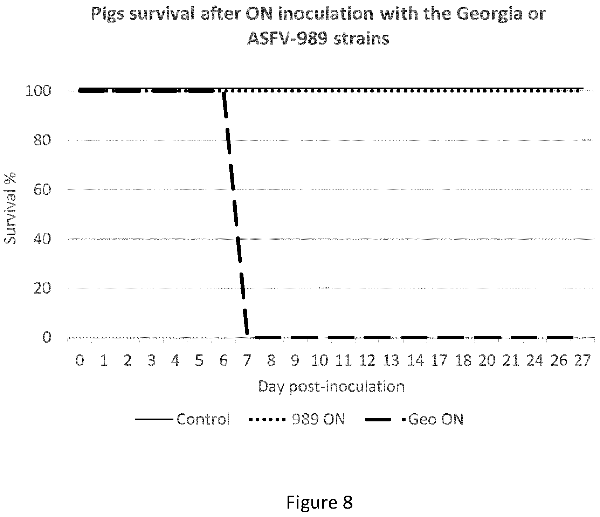

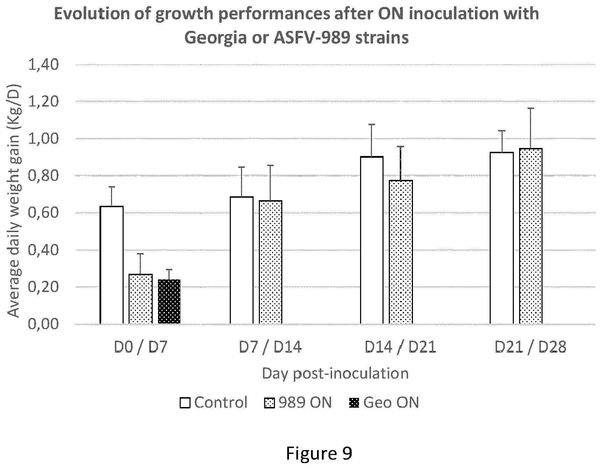

Following ON inoculation of the Georgia strain, the pigs (Geo ON: group F) displayed the same severe symptoms as for IM inoculation with a peak of hyperthermia (41.0±0.4°C) at D4 PI (Figure 7). All the pigs had also to be euthanatized at D6 PI (Figure 8). On the other hand, pigs inoculated ON with the ASFV-989 strain (989 ON: groups B, H and I) developed a lower fever (40.6±0.5°C at D6 PI) (Figure 7), limited symptoms and all the pigs survived the infection (Figure 8). Pigs inoculated ON with the ASFV-989 strain also displayed a drop of growth performances (ADWG between D0/D7 PI: 0.27±0.11 kg/D for 989 ON versus 0.64±0.10 kg/D for control), but only during the first week after inoculation (Figure 9).

### + Virological data

To further characterize the attenuated nature of the ASFV-989 strain, we then evaluated the viremia level for the ASFV-989 strain in comparison to Georgia, for pigs inoculated by IM or ON route during the first week of infection. As depicted in figure 10, the viremia level for the ASFV-989 strain is markedly lower than for the Georgia strain with 100-fold lower viral load for the ASFV-989 strain. For the pigs inoculated IM with the Georgia strain the ASFV viremia at D5 PI was 8.5±0.2 log10 eq HAD₅₀/ml but only 5.9±0.3 log10 eq HAD₅₀/ml for the animals inoculated by the same route with the ASFV-989 strain. Interestingly, the pigs inoculated by ON route with the ASFV-989 strain displayed a lower viremia than those inoculated IM (at D5 PI, 4.7±1.6 log10 eq HAD₅₀/ml for ON versus 5.9±0.3 log10 eq HAD₅₀/ml for IM inoculation route).

To describe more precisely the ASFV viremia profile in pigs inoculated with the ASFV-989 strain (either IM or ON), we followed the viremia for the ASFV-989 strain for up to 100 days with the 989 PCR for the animals of groups B and C. The data shown in figure 11 confirmed the lower level of viremia for ON inoculation route (at D7 PI 6.2±0.3 log10 eq HAD₅₀/ml for IM versus 5.5±0.4 log10 eq HAD₅₀/ML for ON), with an earlier extinction of viremia for ON inoculation route (at D76 PI: 0/4 pigs viremic for ON versus 3/4 still viremic for IM).

### + ASFV specific immune response

### -Antibody response

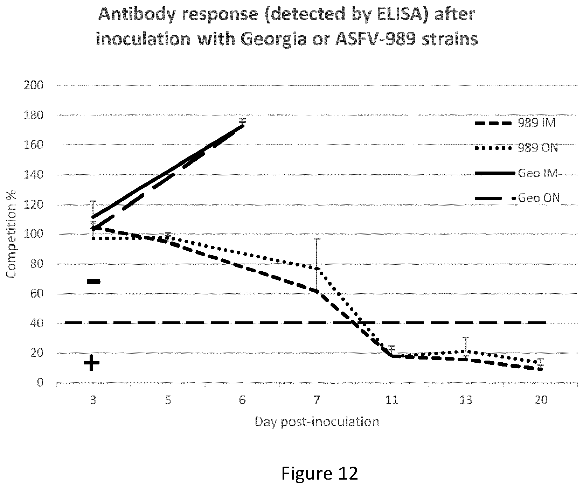

After inoculation of the Georgia strain, the infected pigs did not develop ASF specific antibody before they have to be euthanized. For the ASFV-989 strain inoculated pigs, both ON or IM inoculated pigs developed ASF specific antibodies with a seroconversion occurring between D7 and D11 PI (Figure 12).

### - Cell mediated response

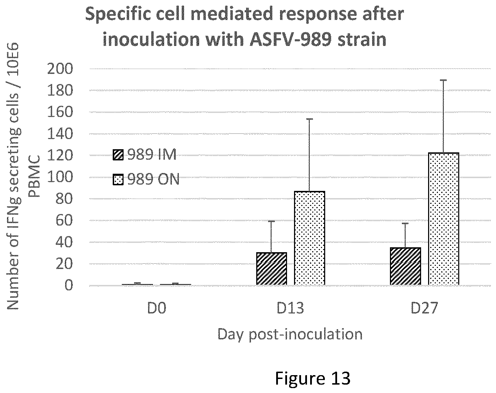

The cell mediated immune (CMI) response specific to ASFV was evaluated by ELISPOT IFNg among pigs inoculated IM or ON with the ASFV-989 strain. A CMI response specific to the ASFV virus was detected as soon as D13 PI and confirmed at D27 PI (Figure 13). The level of the CMI response seemed to be stronger for the ON group (122±67 IFNg SCs / 10E6 PBMC at D27 PI) compared to the IM one (35±23 IFNg SCs / 10E6 PBMC at D27 PI).

### 2.3 Protective effect of ASFV-989 strain immunization toward a challenge with the Georgia strain

Having characterized the immune response induced by the ASFV-989 strain inoculation, we then evaluated if the immunized pigs may be protected against an ASFV challenge with the virulent Georgia strain (Table 1)

### + Clinical and zootechnical data

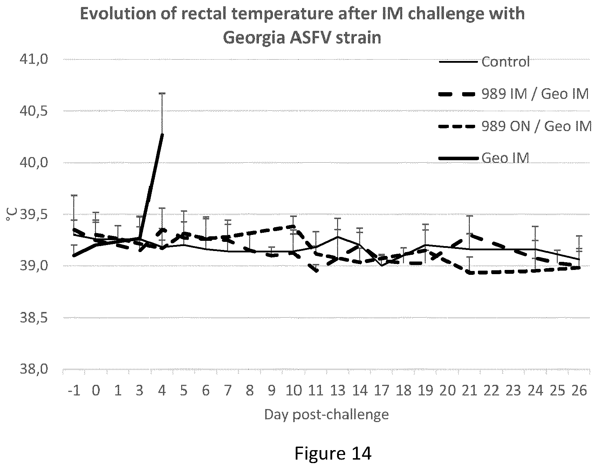

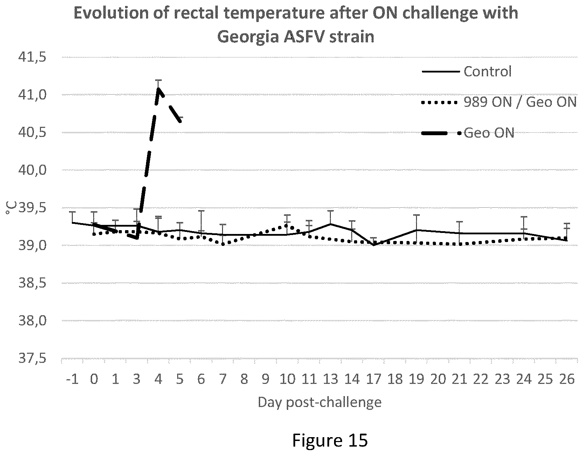

As shown previously, challenge with the Georgia strain by ON or IM route induced a rapid hyperthermia in non-immunized pigs (Figure 14 and 15) with severe symptoms that imposed the compassionate euthanasia of the pigs (groups G and J). On the contrary, none of the pigs immunized with the ASFV-989 strain and challenged with Georgia developed hyperthermia or ASF related symptoms and all survived the Georgia challenge (groups D, H and I).

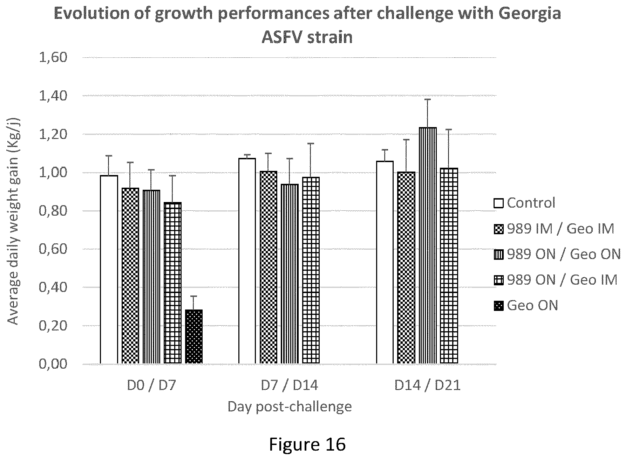

In terms of growth performances, the Georgia challenge had no impact in the ASFV-989 immunized pigs in contrast to non-immunized animals that demonstrated a quick drop of average daily weight gain (Figure 16).

### + Virological data

Having determined that the pigs immunized with the ASFV-989 strain are completely protected against the Georgia challenge at the clinical point of view, we then assessed the effect of ASFV-989 immunization at the virological level. Using both the PCR 505 (detecting the Georgia strain) and the PCR 989 (detecting the ASFV-989 strain) we evaluated the Georgia and ASFV-989 viremia in pigs (previously immunized or not with ASFV-989) challenged with the Georgia strain.

As shown in Table 2 and 3, both group of non-immunized pigs displayed a quick rise of Georgia viremia after challenge (At D5 PC: 8.9±0.2 log10 eq HAD50/ml for Georgia IM and 9.0±0.4 log10 eq HAD50/ml for Georgia ON). In the contrary, the Georgia genome was not detected in the blood of any of the ASFV-989 immunized pigs, whatever the route of immunization or the route of challenge. As shown previously, the PCR 989 results confirmed that the ASFV-989 viremia could be detected for at least 3 weeks after immunization.

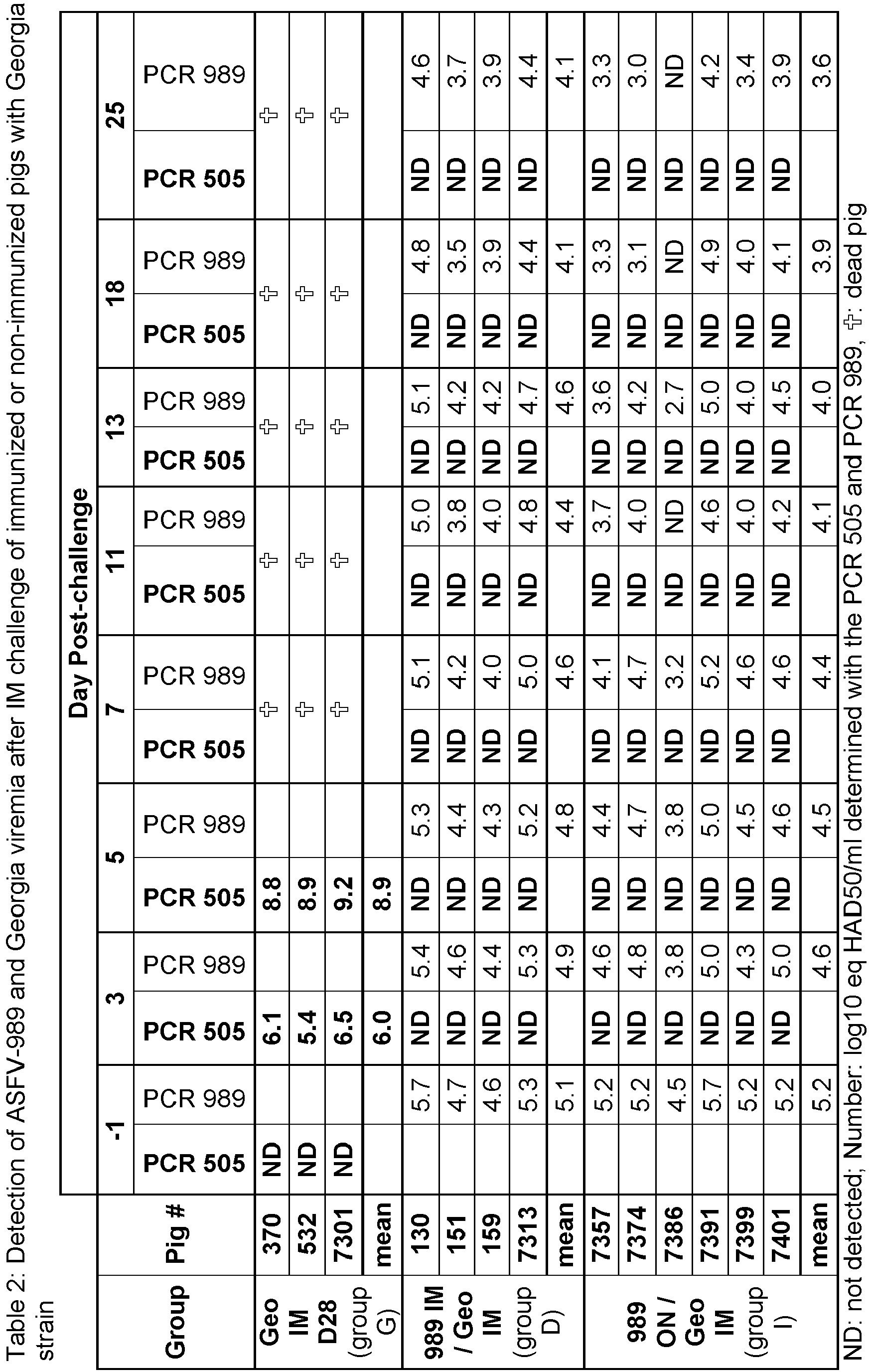

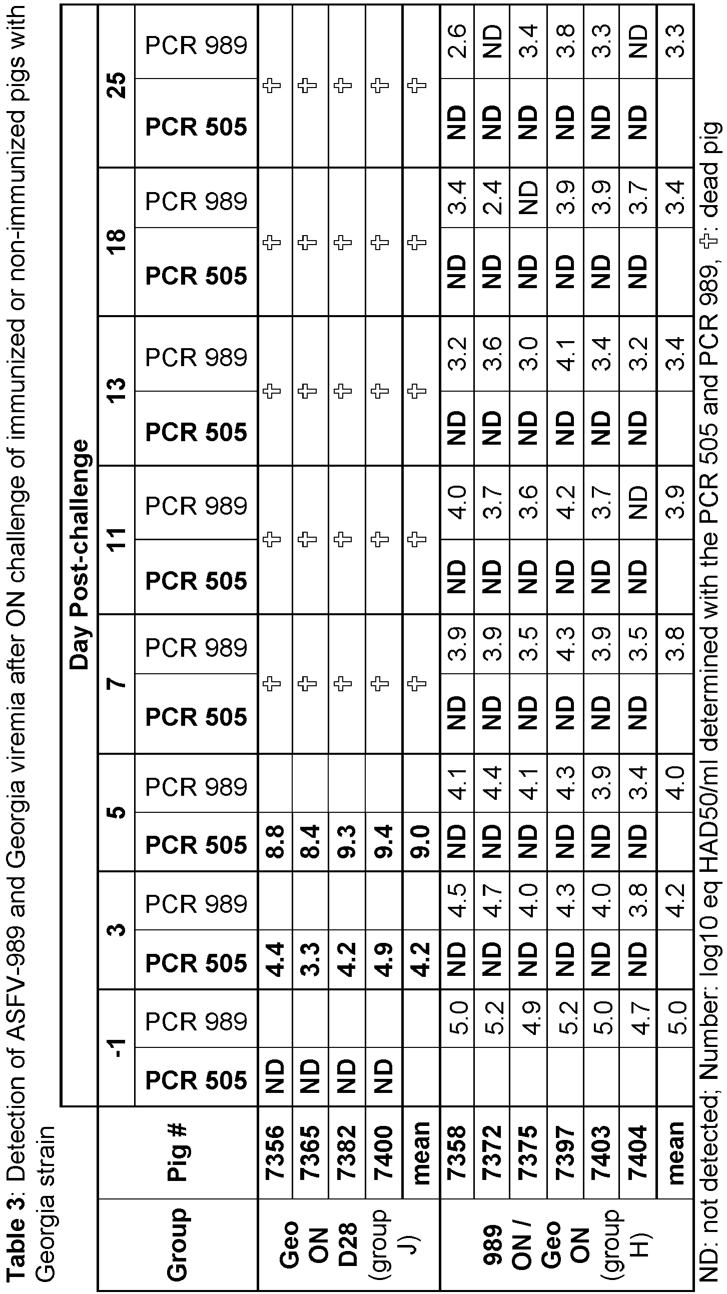

On tissue samples collected at necropsy, the Georgia genome was not detected in any of the ASFV-989 IM immunized pigs challenged by IM route (Table 4). Among the pigs immunized with the ASFV-989 strain by ON route, the Georgia genome was detected in the tissues (Tonsil, spleen or hepato-gastric lymph node) of 4 pigs out of 12 (2 in the group H and 2 in the group I).

**Table 4: Detection of Georgia ASFV genome in tissues of immunized or non-immunized pigs after challenge with Georgia strain**

| **Group** | **Pig #** | **Tonsil** | **Spleen** | **Hepato-gastric LN** |
|---|---|---|---|---|
| **989 IM** / **Geo IM** (group D) | 130 | ND | ND | ND |
| | 151 | ND | ND | ND |
| | 159 | ND | ND | ND |
| | 7313 | ND | ND | ND |
| **989 ON / Geo IM** (group I) | 7357 | ND | ND | ND |
| | 7374 | ND | ND | ND |
| | 7386 | ND | ND | ND |
| | 7391 | ND | ND | ND |
| | 7399 | **27.55** | **36.73** | **41.12** |
| | 7401 | **32.64** | ND | **32.21** |
| **989 ON / Geo ON** (group H) | 7358 | ND | ND | ND |
| | 7372 | ND | ND | ND |
| | 7375 | ND | ND | ND |
| | 7397 | ND | **37.48** | ND |
| | 7403 | ND | ND | ND |
| | 7404 | ND | **43.42** | ND |

| | | | | |
|---|---|---|---|---|
| ND: not detected; Number: Ct value for the PCR 505. Tissues collected during the necropsies performed at D40 PC | | | | |

### Reference List

1. O'Donnell et al.: "African Swine Fever Virus Georgia Isolate Harboring Deletions of MGF360 and MGF505 Genes Is Attenuated in Swine and Confers Protection against Challenge with Virulent Parental Virus", J Virol. 2015 Jun;89(11):6048-56.
2. US20160130562
3. Xuexia Wen et al. (2019) Genome sequences derived from pig and dried blood pig feed samples provide important insights into the transmission of African swine fever virus in China in 2018, Emerging Microbes & Infections, 8:1, 303-306.
4. Bao, J et al. Genome comparison of African swine fever virus China/2018/AnhuiXCGQ strain and related European p72 Genotype II strains. Transbound Emerg Dis. 2019; 66: 1167- 1176.
5. Mazur-Panasiuk N. et al. The first complete genomic sequences of African swine fever virus isolated in Poland. Sci Rep 9, 4556 (2019).
6. Forth JH et al. Comparative Analysis of Whole-Genome Sequence of African Swine Fever Virus Belgium 2018/1. Emerg Infect Dis. 2019 Jun;25(6):1249-1252.
7. Kovalenko, G. et al. Complete Genome Sequence of a Virulent African Swine Fever Virus from a Domestic Pig in Ukraine. Microbiology resource announcements 2019, 8.
8. Rathakrishnan A et al. Production of Recombinant African Swine Fever Viruses: Speeding Up the Process. Viruses. 2020 Jun 5;12(6):615.
9. Carrascosa, A. L. et al. Methods for growing and titrating African swine fever virus: field and laboratory samples. Curr Protoc Cell Biol, 2011. Chapter 26: p. Unit 26 14.
10. Bolger, A.M., M. Lohse, and B. Usadel, Trimmomatic: a flexible trimmer for Illumina sequence data. Bioinformatics, 2014. 30(15): p. 2114-20.
11. Li, H. and R. Durbin, Fast and accurate short read alignment with Burrows-Wheeler transform. Bioinformatics, 2009. 25(14): p. 1754-60.
12. Li, H., et al., The Sequence Alignment/Map format and SAMtools. Bioinformatics, 2009. 25(16): p. 2078-9.
13. Tignon, M., et al., Development and inter-laboratory validation study of an improved new real-time PCR assay with internal control for detection and laboratory diagnosis of African swine fever virus. J Virol Methods, 2011. 178(1-2): p. 161-70.
14. King, K., et al., Protection of European domestic pigs from virulent African isolates of African swine fever virus by experimental immunisation. Vaccine, 2011. 29(28): p. 4593-600

## Claims

1. An attenuated African Swine Fever (ASF) virus, wherein :
• genes MGF 360-12L, 360-13L, 360-14L, 505-2R, 505-3R are deleted or are interrupted or mutated such that the genes are not transcribed and/or translated,
• ORF of ASFV_G_ACD_00520 is deleted or is interrupted or mutated such that it is not transcribed and/or translated, and
• genes MGF 505-1R et 505-4R are truncated,
compared to the genome of the corresponding unattenuated virus.

2. An attenuated ASF virus according to claim 1, wherein:
• genes MGF 360-12L, 360-13L, 360-14L, 505-2R, 505-3R are deleted, and
• ORF of ASFV_G_ACD_00520 is deleted.

3. An attenuated ASF virus according to claim 1, wherein:
• genes MGF 360-12L, 360-13L, 360-14L, 505-2R, 505-3R are interrupted such that the genes are not transcribed and/or translated,
• ORF of ASFV_G_ACD_00520 is interrupted such that it is not transcribed and/or translated.

4. An attenuated ASF virus according to any preceding claim, wherein the interruption comprises deletion or modification of the ATG start codon of the gene; or a modification of a translational start site; or a frame shift; or introduction of one or more stop codons in the open reading frame of the gene.

5. An attenuated ASF virus according to any preceding claim, wherein the remainder of the genome corresponds to the genome from a virulent ASFV virus strain selected among the lineage of Georgia 2007/1, for example Pig/HLJ/2018, ASFV-SY18, China/2018/AnhuiXCGQ, ASFV/POL/2015/Podlaskie, Odintsovo 2/2014, Kashino 04/13, ASFV-Belgium2018/1, ASFV/Kyiv/2016/131, a strain of ASF virus of genotype II or a genetically close ASF virus strain.

6. An attenuated ASF virus according to any preceding claim, wherein the remainder of the genome corresponds to the genome from the virulent ASFV virus strain Georgia 2007/1.

7. An attenuated ASF virus according to any preceding claim, which does not contain any reporter gene.

8. A vaccine comprising an attenuated ASF virus according to any preceding claim.

9. A vaccine according to claim 8, which comprises different strains of said attenuated ASF virus.

10. An attenuated ASF virus according to anyone of claims 1 to 7, or a vaccine according to anyone of claims 8 to 9, for use in preventing African Swine Fever in a subject.

11. An attenuated ASF virus or a vaccine for use according to claim 10, by inducing in the subject a protective immune response against the virulent ASFV virus strain Georgia 2007/1 or against other genetically close ASF virus strains among the Georgia 2007/1 lineage.

12. An attenuated ASF virus or a vaccine for use according to claim 10 or 11, wherein said attenuated ASF virus or said vaccine is administered by a route selected among oronasal route and parenteral route, especially intra-muscular route.

13. An attenuated ASF virus or a vaccine for use according to claim 10 to 12, wherein the subject is a pig or a wild-boar, and/or wherein the vaccine is administered following a prime-regime.

14. An in-vitro method for obtaining an attenuated ASF virus according to anyone of claims 1 to 7, which comprises the step of :
• Deleting or interrupting genes MGF 360-12L, 360-13L, 360-14L, 505-2R, 505-3R so that the genes are not transcribed and/or translated,
• Deleting or interrupting ORF of ASFV_G_ACD_00520 so it is not transcribed and/or translated,
• Truncating genes MGF 505-1R et 505-4R,
compared to the genome of the corresponding unattenuated virus.

15. The method according to claim 14, wherein :
• genes MGF 360-12L, 360-13L, 360-14L, 505-2R, 505-3R are deleted, and
• ORF of ASFV_G_ACD_00520 is deleted.

16. The method according to claim 14, wherein :
• genes MGF 360-12L, 360-13L, 360-14L, 505-2R, 505-3R are interrupted such that the genes are not transcribed and/or translated,
• ORF of ASFV_G_ACD_00520 is interrupted such that it is not transcribed and/or translated.

17. A method according to any of claims 14 to 16, wherein the interruption comprises: deletion or modification of the ATG start codon of the genes or a modification of a translational start site; or a frame shift; or introduction of one or more stop codons in the open reading frame of the gene.

18. An in-vitro method for obtaining an attenuated ASF virus according to anyone of claims 1 to 7, which comprises at least one step of thermal-attenuation of a virulent ASFV virus strain selected among Georgia 2007/1, Pig/HLJ/2018, a strain of ASF virus of genotype II or a genetically close ASF virus strain, and amplification by inoculation of Specific-Pathogen-Free pigs and selecting said attenuated ASF virus.

19. An in vitro method for the differential detection, in a sample, of an attenuated ASF virus according to anyone of claims 1 to 7, and of the corresponding non-attenuated ASF virus, which comprises carrying out differential amplification of the nucleic acids strands by PCR with sets of primers / probes specific for each strain, wherein primers / probe of sequences:
Del_Fow TGCTTTCAAGCCTACAACTCC (SEQ ID NO: 4);
Del_Rev ATTCTCAGGGCCTCATTGGT (SEQ ID NO: 5) and
Del_Probe AGCGATCCTTTGGCTGCCACC (SEQ ID NO: 6)
allows the specific amplification of the attenuated ASF virus, whereas the primers / probe of sequences :
505_3R_L1 TGGCAAGATCATGGTTCCCT (SEQ ID NO: 7),
505_3R_R1 ATCTGCCTCCCATGACAACA (SEQ ID NO: 8) and
505_3R_P1 CCCTTCCGATGCTGCTACTTTGAGTGC (SEQ ID NO: 9)
allows the specific amplification of the corresponding non-attenuated ASF virus.
